(19) Europäisches Patentamt
European Patent Office
Office européen des brevets

(11) Veröffentlichungsnummer: **0 054 876**
**A1**

# (12) EUROPÄISCHE PATENTANMELDUNG

(21) Anmeldenummer: **81110409.0**

(22) Anmeldetag: **14.12.81**

(51) Int. Cl.³: **C 07 D 239/06**, C 08 G 18/20

(30) Priorität: **24.12.80 DE 3049131**

(43) Veröffentlichungstag der Anmeldung: **30.06.82 Patentblatt 82/26**

(84) Benannte Vertragsstaaten: **BE DE FR GB IT NL**

(71) Anmelder: **BAYER AG, Zentralbereich Patente, Marken und Lizenzen, D-5090 Leverkusen 1, Bayerwerk (DE)**

(72) Erfinder: **Rasshofer, Werner, Dr., Wolfskaul 10, D-5000 Köln 80 (DE)**
Erfinder: **Grögler, Gerhard, Dr., von-Diergardt-Strasse 46, D-5090 Leverkusen 1 (DE)**
Erfinder: **Kopp, Richard, Dr., Wolfskaul 12, D-5000 Köln 80 (DE)**

## (54) Tetrahydropyrimidine, ein Verfahren zu ihrer Herstellung und ihre Verwendung als Katalysatoren bei der Herstellung von Polyurethankunststoffen.

(57) Die Erfindung betrifft Tetrahydropyrimidine der Formel

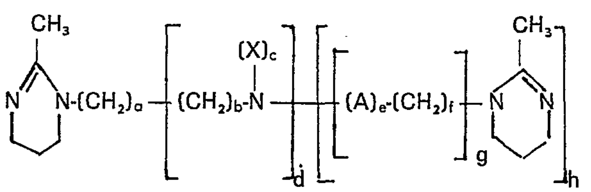

in der A einen divalenten, $C_1$-$C_{17}$-Alkanrest, $C_5$-$C_{10}$-Cycloalkanrest oder einen Dicyclohexyl-$C_1$-$C_4$-alkanrest, die durch einen Phenylrest, eine Cyangruppe oder einen Halogenrest substituiert sein können und wobei der $C_1$-$C_{17}$-Alkanrest in der Kette durch -NH-COO-, -O- und/oder -S-Einheiten unterbrochen sein kann,

X einen $C_1$-$C_{16}$-Alkylrest oder einen $C_5$-$C_7$-Cycloalkylrest, die durch einen Phenylrest, eine Cyangruppe, eine Hydroxygruppe oder einen Halogenrest substituiert sein können, oder, wenn h = 0 ist, zusammen einen Ring mit zusammen 3-6 Ring-C-Atomen bilden, von denen ein oder zwei C-Atome durch -O-, -S-, -NH- oder -NR³- ersetzt sein können, wobei

R³ für $C_1$-$C_6$-Alkyl oder ω-Hydroxy-$C_1$-$C_6$-Alkyl steht,
a, b, f, g jeweils ganze Zahlen von 1 bis 16,
c 1 oder 2,
h 0 oder 1,
wobei $\Sigma c + h = 2$ ist
d 0 oder eine ganze Zahl von 1 bis 10 und
e 0 oder 1
bedeuten, ihre Herstellung aus N-substituierten Bis-propylendiaminen der Formel

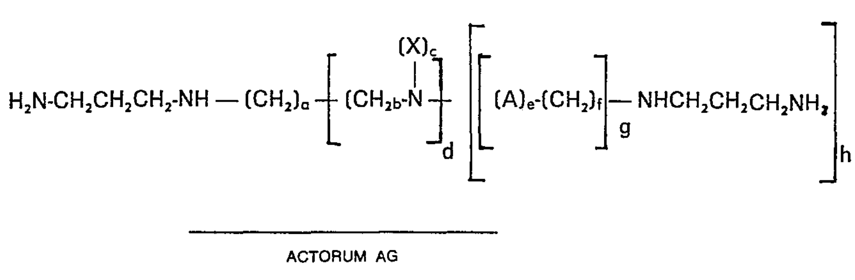

EP 0 054 876 A1

in der
A, X, a, b, c, d, e, f, g und h die oben angegebenen Bedeutungen haben, durch Umsetzung mit einem Acetessigsäurederivat der Formel

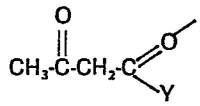

in der
Y für $-OR^4$, $-NHR^4$ oder $-NR^4_2$ und
$R^4$ für einen $C_6$-$C_{10}$-Aryl-, $C_1$-$C_{17}$-Alkyl- oder $C_5$-$C_7$-Cycloalkylrest
steht,
in Abwesenheit von Katalysatoren bei einer Reaktionstemperatur von 0-80°C und kontinuierlicher Entfernung des dabei entstehenden Wassers,
sowie ihre Verwendung als Katalysatoren bei der Herstellung von Polyurethankunststoffen.

0054876

BAYER AKTIENGESELLSCHAFT 5090 Leverkusen, Bayerwerk

Zentralbereich
Patente, Marken und Lizenzen GM/m-c

# Tetrahydropyrimidine, ein Verfahren zu ihrer Herstellung und ihre Verwendung als Katalysatoren bei der Herstellung von Polyurethankunststoffen

Die Erfindung betrifft neue Tetrahydropyrimidine, ein Verfahren zu ihrer Herstellung und ihre Verwendung als Katalysatoren bei der Herstellung von Polyurethankunststoffen einschließlich Polyurethanschaumstoffen.

Es sind bereits viele Amine als Katalysatoren bei der Herstellung von Polyurethanen durch Umsetzung von Polyolen mit Polyisocyanaten verwendet worden (vgl. K.C. Frisch, L.P. Rennao, "Catalysis in Isocyanate Reactions" in J. Macromol. Sci. - Revs. Macromol. Chem. Soc. C 5 (1), 103-150).

Bei den aktivsten Aminen, die in der Regel tertiär gebundenen Stickstoff enthalten, z.B. 1,4-Diazabicyclooctan (2.2.2) (Triethylendiamin, Dabco®), sind Konzentrationen von 0,04 bis 0,5 Gew.-Teilen, bezogen auf eingesetztes Polyol, erforderlich. Bei anderen Aminen werden noch erheblich größere Mengen benötigt, ebenso, wenn aliphatische Isocyanate eingesetzt werden.

Selbst diese geringen Mengen an Aminkatalysator zeigen indes oftmals schwerwiegende Nachteile. Da es sich in der Regel um tertiäre Amine handelt, die nicht einbaufähig sind, ist die Folge, daß noch lange Zeit nach der Herstellung ein allgemein als störend empfundener Geruch auftritt.

Besonders trifft das für Gegenstände des täglichen Bedarfs zu, wie Polsterwaren, Einrichtungen des Automobilbaus, Schuhen, Möbeln usw. Auch sind diese Amine häufig der Grund für das Vergilben heller Leder- oder Kunststoffoberflächen.

Ein weiterer Nachteil solcher nichteinbaubarer Aminkatalysatoren ist, daß sie oft zum Ausschwitzen neigen oder durch andere physikalische und/oder chemische Prozesse an die Oberfläche transportiert werden. So kann z.B. auf einem, mit Diazabicycloundecen katalysierten Polyurethanmaterial, wie es zur Herstellung von Lenkrädern und Kopfstützen Verwendung findet, ein weißer Belag auftreten, in dem Diazabicycloundecen aufgefunden wird.

Desgleichen beobachtet man häufig, wenn Triethylendiamin als Katalysator bei der Herstellung von Polyurethan-Schuhsohlen Verwendung findet, bei hellen Oberledern Verfärbungen, die es unmöglich machen, die Sohle direkt an den Schuhschaft anzuschäumen. Diese Verfärbung wird durch bei der Verschäumung austretendes Triethylendiamin hervorgerufen. Desgleichen wird ein solcher Effekt bei der Verwendung von nicht einbaufähigen Tetrahydropyrimidinen als Katalysator gefunden.

Acyclische Amidine als Katalysatoren bei der Herstellung von Polyurethanen aus aliphatischen Isocyanaten beschreibt die DE-OS 1 950 262. Mit der DE-OS 2 737 671 wird der Einsatz von Kombinationen aus cyclischen Amidinen, die Tetrahydropyrimidin- oder Imidazolin-Strukturen besitzen, und Metallsalzen als Katalysatoren für die Herstellung von Polyurethanen gelehrt.

Die veröffentlichte japanische Patentanmeldung 7 102 672 beschreibt die Verwendung von Tetrahydropyrimidinen zur Katalysierung der Urethanisierungsreaktion. Bicyclische Amidine als Polyurethan-Katalysatoren werden in der DE-OS 1 745 418 beschrieben.

Alle diese Katalysatoren entsprechen indessen noch nicht den in der Praxis gestellten Forderungen nach einer hohen katalytischen Aktivität und nach Geruchsfreiheit des erhaltenen Kunststoffs.

Die DE-OS 2 601 082 lehrt schon die Herstellung von Polyurethanen unter Verwendung von einbaufähigen Katalysatoren vom Amidintyp; allerdings weisen die dort genannten Aminopyridine nur für die Herstellung von Polyurethanen aus aromatischen Polyisocyanaten eine ausreichende Aktivität auf. Für die Herstellung von

Polyurethanen aus aliphatischen Polyisocyanaten ist die katalytische Wirksamkeit der in dieser DE-OS genannten Aminopyridine zu gering. Die Aminopyridine besitzen somit eine für die praktischen Ansprüche oft nicht ausreichende Anwendungsbreite als Katalysatoren bei der Herstellung von Polyurethanen.

Mit der vorliegenden Erfindung gelingt es nun unerwarteterweise, die aus dem Stand der Technik bekannten Nachteile zu vermeiden. Die erfindungsgemäß zu verwendenden Tetrahydropyrimidine stellen nämlich hervorragende Katalysatoren für die Herstellung von Polyurethanen, sowohl aus aliphatischen als auch aus aromatischen Polyisocyanaten dar, wobei die mit ihrer Hilfe hergestellten Polyurethankunststoffe nunmehr keinen wahrnehmbaren Amingeruch mehr aufweisen.

Die erfindungsgemäßen Tetrahydropyrimidine haben einen sehr viel niederen Dampfdruck als die cyclischen Amidine nach DE-OS 1 950 262, der bicyclischen Amidine nach DE-OS 2 737 671 und der Tetrahydropyrimidine nach der japanischen Patentanmeldung 7 102 672.

Sie weisen darüber hinaus als Katalysatoren folgende Vorteile auf:

1. Sie sind im allgemeinen besonders gut verträglich mit der PU-Matrix,

2. sie haben vielfach einen besonders hohen Gehalt an katalytisch wirksamen Gruppen im Molekül,

3. sie sind hydrolysestabiler als die bisher bekannten monocyclischen Tetrahydropyrimidine.

Gegenstand der vorliegenden Erfindung sind somit neue Tetrahydropyrimidine der allgemeinen Formel I

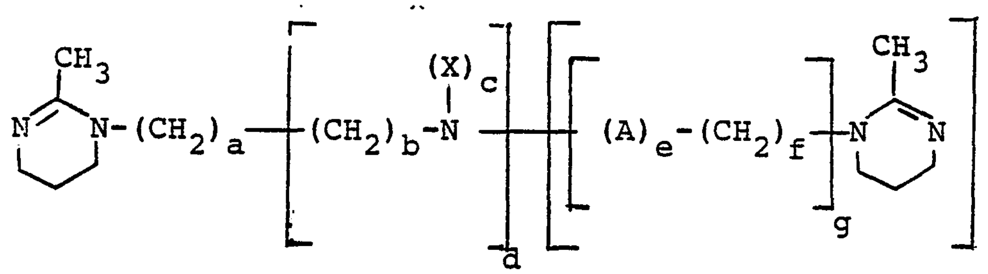, I

in der

A einen divalenten geradkettigen oder verzweigten $C_1$-$C_{17}$-Alkanrest oder $C_5$-$C_{10}$-Cycloalkanrest oder einen Dicyclohexyl-$C_1$-$C_4$-alkanrest, die durch einen Phenylrest, eine Cyangruppe oder einen Halogenrest substituiert sein können und wobei der $C_1$-$C_{17}$-Alkanrest in der Kette durch -NH-COO-, -O- und/oder -S-Einheiten unterbrochen sein kann,

X einen geradkettigen oder verzweigten $C_1$-$C_{16}$-Alkylrest oder einen $C_5$-$C_7$-Cycloalkylrest, die durch einen Phenylrest, eine Cyangruppe, eine Hydroxygruppe oder einen Halogenrest substituiert sein können, oder wenn h = O ist, zusammen einen Ring mit zusammen 3-6 Ring-C-Atomen bilden, von denen ein oder zwei C-Atome durch -O-, -S-, -NH- oder $-NR^3-$ ersetzt sein können, wobei

$R^3$ für $C_1$-$C_6$-Alkyl oder $\omega$-Hydroxy-$C_1$-$C_6$-Alkyl steht,

a, b, f, g jeweils ganze Zahlen von 1 bis 16,

c 1 oder 2

h 0 oder 1,

wobei $\sum$ c + h = 2 ist,

d 0 oder eine ganze Zahl von 1 bis 10 und

e 0 oder 1

bedeuten.

Erfindungsgemäß bevorzugt sind neue Tetrahydropyrimidine der allgemeinen Formel II

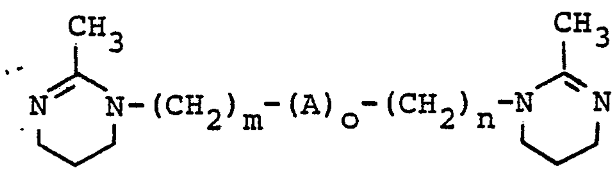

, II

in der

A einen divalenten, geradkettigen oder verzweigten $C_1$-$C_{17}$-Alkanrest oder einen divalenten $C_5$-$C_{10}$-Cycloalkanrest oder einen Dicyclohexyl-$C_1$-$C_4$-alkanrest, die durch einen Phenylrest, eine Cyangruppe oder einen Halogenrest

substituiert sein können und wobei der $C_1$-$C_{17}$-Alkanrest in der Kette durch -NH-COO-, -O- und/oder -S-Einheiten unterbrochen sein kann,

m eine ganze Zahl von 1 bis 16,

n eine ganze Zahl von 1 bis 16 und

o 0 oder 1

bedeuten.

Erfindungsgemäß bevorzugt sind ferner neue Tetrahydropyrimidine der allgemeinen Formel III

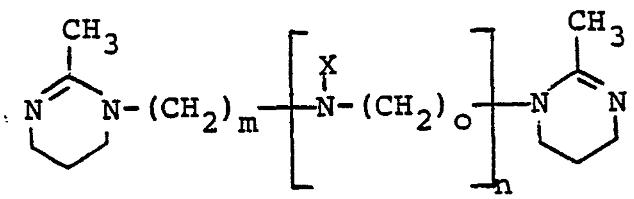

, III

in der

X für einen geradkettigen oder verzweigten $C_1$-$C_{16}$-Alkylrest oder einen $C_5$-$C_7$-Cycloalkylrest, die durch einen Phenylrest, eine Cyangruppe oder einen Halogenrest substituiert sein können,

m, o jeweils eine ganze Zahl von 2 bis 10 und

n eine ganze Zahl von 1 bis 16

bedeuten.

Erfindungsgemäß bevorzugt sind auch Tetrahydropyrimidine der allgemeinen Formel IV

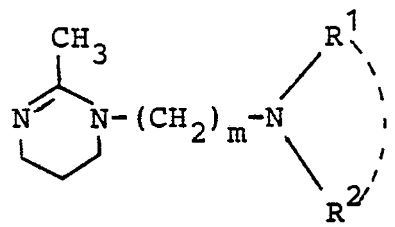

, IV

in der

m eine ganze Zahl von 2 bis 10 ist und

$R^1$, $R^2$ unabhängig voneinander einen geradkettigen, oder verzweigten $C_1$-$C_{10}$-Alkylrest oder einen $C_5$-$C_7$-Cycloalkylrest, die durch einen Phenylrest, eine Cyangruppe, eine Hydroxylgruppe oder ein Halogenatom substituiert sein können,

bedeuten, oder

zusammen einen Ring mit zusammen 3-6 Ring-C-Atomen bilden, von denen ein oder zwei C-Atome durch -O-,

-S-, -NH- oder $-NR^3-$ ersetzt sein können, wobei

$R^3$ für $C_1$-$C_6$-Alkyl oder $\omega$-Hydroxy-$C_1$-$C_6$-alkyl steht.

Zur Modifizierung der katalytischen Aktivität der erfindungsgemäßen Tetrahydropyrimidin-Einheiten enthaltenden Verbindungen ist es oft zweckmäßig, sie in Form ihrer Salze, Additionsverbindungen und Komplexe einzusetzen. Dafür kommen Protonsäuren wie (an)organische Säuren und andere acide Verbindungen in Frage, bzw. prinzipiell alle Verbindungen, deren Basenstärke kleiner als die der Tetrahydropyrimidine ist.

Weiterhin kommen Lewissäuren in Frage, die als koordinativ ungesättigte Verbindungen mit Elektronendonoren reagieren. Diese Modifizierung führt auch zum Teil zu einem weiter erhöhten Hydrolyseschutz für die erfindungsgemäßen Tetrahydropyrimidin-Einheiten enthaltenden Verbindungen.

Gegenstand der vorliegenden Erfindung sind also ferner Salze, Additionsverbindungen und Komplexe der erfindungsgemäßen Tetrahydropyrimidine mit Protonsäuren und Lewissäuren.

Die vorliegende Erfindung betrifft ferner ein Verfahren zur Herstellung von Tetrahydropyrimidinen der allgemeinen Formel I

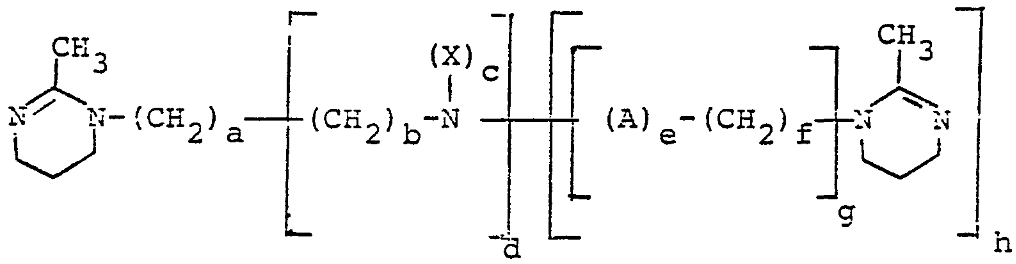, I

in der

A einen divalenten, geradkettigen oder verzweigten $C_1$-$C_{17}$-Alkanrest oder einen divalenten $C_5$-$C_{10}$-Cycloalkanrest oder einen Dicyclohexyl-$C_1$-$C_4$-alkanrest, die durch einen Phenylrest, eine Cyangruppe oder einen Halogenrest substituiert sein können und wobei der $C_1$-$C_{17}$-Alkanrest in der Kette durch -NH-COO-, -O- und/oder -S-Einheiten unterbrochen sein kann,

X einen geradkettigen oder verzweigten $C_1$-$C_{16}$-Alkylrest oder einen $C_5$-$C_7$-Cycloalkylreste, die durch einen Phenylrest, eine Cyangruppe, eine Hydroxygruppe oder einen Halogenrest substituiert sein können, oder wenn h = O ist, zusammen einen Ring mit zusammen 3-6 Ring-C-Atomen bilden, von denen ein oder zwei C-Atome durch -O-, -S-, -NH- oder $-NR^3-$ ersetzt sein können, wobei

$R^3$ für $C_1$-$C_6$-Alkyl oder $\omega$-Hydroxy-$C_1$-$C_6$-Alkyl steht,

a, b, f, g jeweils ganze Zahlen von 1 bis 16,

c 1 oder 2,

h 0 oder 1,

wobei $\Sigma$ c+h = 2 ist,

d 0 oder eine ganze Zahl von 1 bis 10 und

e 0 oder 1

bedeuten,

durch Umsetzung von N-substituierten Propylendiaminen mit Acetessigsäurederivaten, dadurch gekennzeichnet, daß man als N-substituierte Propylendiamine Verbindungen der allgemeinen Formel V

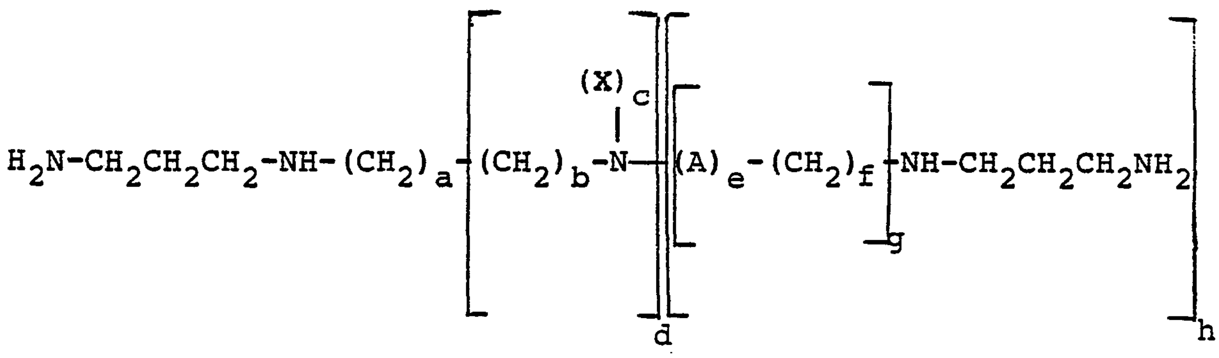

V

in der

A, X, a, b, c, d, e, f, g und h die oben angegebenen Bedeutungen haben, mit einem Acetessigsäurederivat der allgemeinen Formel VI

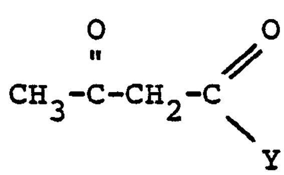

, VI

in der

Y für $-OR^4$, $-NHR^4$ oder $-NR^4_2$ und

$R^4$ für einen $C_6$-$C_{10}$-Aryl, $C_1$-$C_{17}$-Alkyl- oder $C_5$-$C_7$-Cycloalkylrest steht,

in Abwesenheit von Katalysatoren bei einer Reaktionstemperatur von 0-80°C umsetzt und dabei das bei der Reaktion entstehende Wasser kontinuierlich entfernt.

Die Erfindung betrifft auch ein Verfahren zur Herstellung von Tetrahydropyrimidinen der allgemeinen Formel II

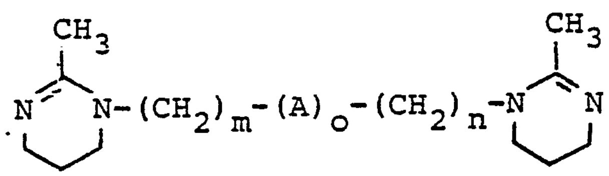

, II

in der

A einen divalenten, geradkettigen oder verzweigten $C_1$-$C_{17}$-Alkanrest oder einen divalenten $C_1$-$C_{10}$-Cycloalkanrest oder einen Dicyclohexyl-$C_1$-$C_4$-alkanrest, die durch einen Phenylrest, eine Cyangruppe oder einen Halogenrest substituiert sein können und wobei der $C_1$-$C_{17}$-Alkanrest in der Kette durch -NH-COO-, -O- und/oder -S-Einheiten unterbrochen sein kann,

m eine ganze Zahl von 1 bis 16

n eine ganze Zahl von 1 bis 16

o 0 oder 1

bedeuten,

durch Umsetzung von N-substituierten Bis-propylendiaminen mit Acetessigsäurederivaten, dadurch gekennzeichnet, daß man als N-substituierte Bis-propylendiamine Verbindungen der allgemeinen Formel

$$H_2NCH_2CH_2CH_2NH(CH_2)_m-(A)_o-(CH_2)_n-NHCH_2CH_2CH_2NH_2,$$

in der
A, m, n und o die oben genannten Bedeutungen haben, mit einem Acetessigsäurederivat der allgemeinen Formel VI

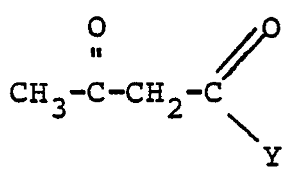
, VI

in der
Y für $-OR^4$, $-NHR^4$ oder $-NR^4_2$ und

$R^4$ für einen $C_6$-$C_{10}$-Aryl-, $C_1$-$C_{17}$-Alkyl- oder $C_5$-$C_7$-Cycloalkylrest steht,

in Abwesenheit von Katalysatoren bei einer Reaktionstemperatur von 0-80°C umsetzt und dabei das bei der Reaktion entstehende Wasser kontinuierlich entfernt.

Die Erfindung betrifft auch ein Verfahren zur Herstellung von Tetrahydropyrimidinen der allgemeinen Formel III

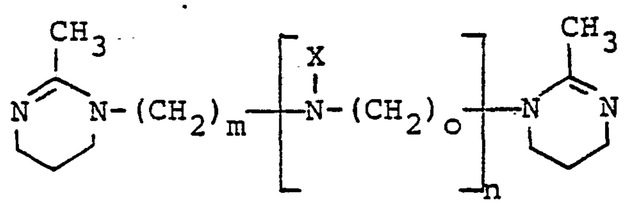

, III

in der

X einen geradkettigen oder verzweigten $C_1$-$C_{16}$-Alkylrest oder einen $C_5$-$C_7$-Cycloalkylrest, die durch einen Phenylrest, eine Cyangruppe oder einen Halogenrest substituiert sein können

m, o jeweils eine ganze Zahl von 2 bis 10 und

n eine ganze Zahl von 1 bis 6

bedeuten,

durch Umsetzung von N-substituierten Bis-propylendiaminen mit Acetessigsäurederivaten, dadurch gekennzeichnet, daß man als N-substituierte Bis-propylendiamine Verbindungen der allgemeinen Formel

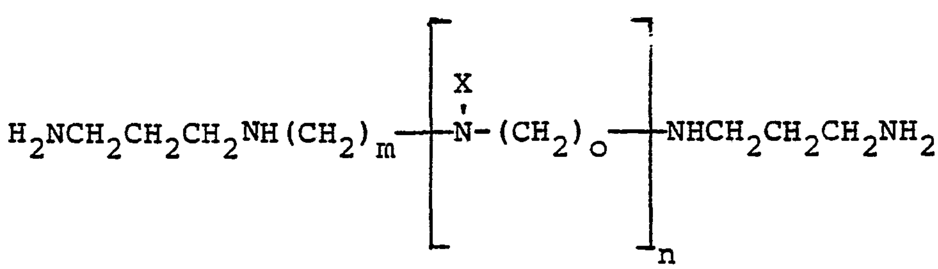

in der
X, m, n und o die oben angegebenen Bedeutungen haben, mit einem Acetessigsäurederivat der allgemeinen Formel VI,

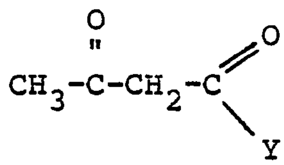

, VI

in der
Y für $-OR^4$, $-NHR^4$ oder $-NR^4_2$ und

$R^4$ für einen $C_6$-$C_{10}$-Aryl-, $C_1$-$C_{17}$-Alkyl- oder $C_5$-$C_7$-Cycloalkylrest steht,

in Abwesenheit von Katalysatoren bei einer Reaktionstemperatur von 0-80°C umsetzt und dabei das bei der Reaktion entstehende Wasser kontinuierlich entfernt.

Die vorliegende Erfindung betrifft weiter ein Verfahren zur Herstellung von Tetrahydropyrimidinen der allgemeinen Formel IV

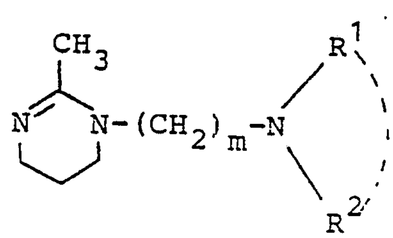

, IV

in der

m eine ganze Zahl von 2 bis 10 ist und

$R^1$, $R^2$ unabhängig voneinander einen geradkettigen oder verzweigten $C_1$-$C_{10}$-Alkylrest oder einen $C_5$-$C_7$-Cycloalkylrest, die durch einen Phenylrest, eine Cyangruppe, eine Hydroxylgruppe oder ein Halogenatom substituiert sein können,

bedeuten, oder

zusammen einen Ring mit zusammen 3-6 Ring-C-Atomen bilden, von denen ein oder zwei C-Atome durch -O-, -S-, -NH- oder $NR^3$- ersetzt sein können, wobei $R^3$ für $C_1$-$C_6$-Alkyl, ω-Hydroxy-$C_1$-$C_6$-alkyl steht,

durch Umsetzung von N-substituierten Bis-propylendiaminen und Acetessigsäurederivaten, dadurch gekennzeichnet, daß man als N-substituierte Bispropylendiamine Verbindungen der allgemeinen Formel

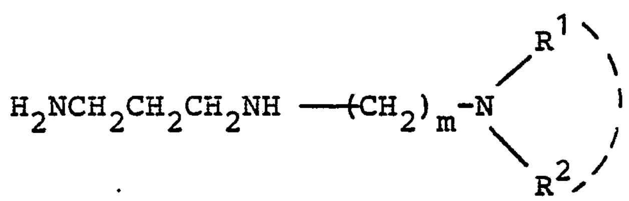

,

in der

m, $R^1$ und $R^2$ die oben genannten Bedeutungen haben, mit einem Acetessigsäurederivat der allgemeinen Formel VI

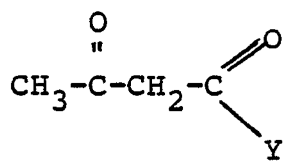 , VI

in der

Y für $-OR^4$, $-NHR^4$ oder $-NR^4_2$ und

$R^4$ für einen $C_6$-$C_{10}$-Aryl-, $C_1$-$C_{17}$-Alkyl- oder $C_5$-$C_7$-Cycloalkylrest steht,

in Abwesenheit von Katalysatoren bei einer Reaktionstemperatur von 0-80°C umsetzt und dabei das bei der Reaktion entstehende Wasser kontinuierlich entfernt.

Die Erfindung betrifft schließlich auch die Verwendung der oben genannten Tetrahydropyrimidine als Katalysatoren bei der Herstellung von Polyurethankunststoffen durch Umsetzung von Polyisocyanaten mit mindestens zwei gegenüber Isocyanaten aktive Wasserstoffatome aufweisende Verbindungen vom Molekulargewicht 400 bis 10 000, gegebenenfalls in Gegenwart von Verbindungen mit mindestens zwei gegenüber Isocyanaten reaktionsfähigen Wasserstoffatomen und einem Molekulargewicht von 32 bis 400 als Kettenverlängerungsmittel und gegebenenfalls in Gegenwart von Treibmitteln und weiteren Hilfs- und Zusatzstoffen.

Es sind schon eine Reihe anderer Verfahren zur Herstellung von 1-Alkyl-2-methyl-tetrahydropyrimidinen bekannt geworden, so beispielsweise die Umsetzung von N-Alkyl-propylen-diaminen mit offenkettigen Imidsäureestern oder Amidinen (A.Pinner, Die Chemie der Imidoether und ihrer Derivate, R. Oppenheim, Berlin, 1892), die Umsetzung von N-Alkyl-propylen-diamintoluolsulfonsäure-salzen mit Carbonsäurenitrilen (J.Chem.Soc. 1947, 497) die Hydrierung von N-Acylamino-nitrilen, wobei die gebildeten N-Acyl-N-Alkyl-propylen-diamine unter den Reaktionsbedingungen zu 1-Alkyl-2-methyl-tetrahydropyrimidinen dehydratisiert werden (J.Am.Chem.Soc. 71, 2350 (1949)) und die Umsetzung von N-Alkyl-propylen-diaminen mit Oxazolinen (DOS 2 154 948).

Viele dieser Verfahren haben jedoch den Nachteil, daß die Umsetzung nicht vollständig abläuft und insbesondere große Mengen unerwünschter Nebenprodukte gebildet werden. Die Reaktionstemperatur bei diesen Herstellungsverfahren beträgt durchweg 100 - 200°C und es wird in Gegenwart von Katalysatoren, beispielsweise sauren Verbindungen (Salzsäure, Toluolsulfonsäure) oder Metallverbindungen (Ni, Co, Cu) gearbeitet.

Weiterhin ist die Umsetzung von N-Ethyl-propylen-diamin mit Acetessigsäureethylester (Chem.Ber. 98, 3652 (1965)) in Gegenwart von Toluolsulfonsäure bei Reaktionstemperaturen von 210°C bekannt. Dieses Verfahren ist an einem 0,2 molaren Reaktionssatz beschrieben, der jedoch nicht auf technische Maßstäbe übertragen werden kann.

0054876

Tetrahydropyrimidine sind als cyclische Amidine außerordentlich hydrolyseanfällig, so daß bei der genannten Temperatur und in Gegenwart von sauren Verbindungen wie Toluolsulfonsäure das cyclische Amidin durch das bei der Kondensation gebildete Wasser sofort verseift wird. Das durch Verseifung entstandene N-Acyl-N-ethyl-propylendiamin kann zwar unter den bei der destillativen Aufarbeitung herrschenden Bedingungen wieder unter Wasserabspaltung langsam recyclisiert werden, was bei einem 0,2 molaren Ansatz in durchaus akzeptablem Zeitraum realisierbar ist, bei einer technischen Durchführung dieses Verfahren in Ansätzen von 100 kg und darüber jedoch zu einer völlig unbefriedigenden Raum-Zeit-Ausbeute führt. Die Aufarbeitung des anfallenden ternären Gemisches aus Tetrahydropyrimidin, seines Verseifungsproduktes N-Acyl-N-alkylpropylen-diamin und Wasser gestaltet sich sehr aufwendig und erfordert eine zeitraubende und umständliche Destillationstechnik. Darüber hinaus erhält man in Folge der thermischen Belastung der Reaktionsmischung einen sehr hohen Anteil an nicht brauchbaren Rückständen, was die Ausbeute an Reinprodukt wesentlich erniedrigt.

Weiterhin ist in Synthesis 1972, Nr. 1, S. 37 die Herstellung von N-substituierten Tetrahydropyrimidinen aus N-Alkylpropandiaminen und $\Delta^2$-Oxazolinen in dem für industrielle Zwecke ungenügenden Ausbeutenbereich von 19-73 % beschrieben.

Der vorliegenden Erfindung liegt daher u.a. die Aufgabe zugrunde, ein Verfahren zu entwickeln, welches erlaubt, Tetrahydropyrimidine der allgemeinen Formeln I, II, III und IV mit hoher Ausbeute und hohem Reinheitsgrad herzustellen.

Überraschenderweise wurde nun gefunden, daß das in DE-AS 2 439 550 beschriebene Verfahren zur Herstellung von Tetrahydropyrimidinen der allgemeinen Formel

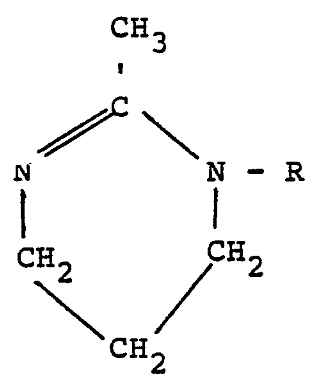
,

in der

R einen geradkettigen, alkylverzweigten oder cyclischen, gesättigten aliphatischen Kohlenwasserstoffrest mit insgesamt 1-17 C-Atomen, der durch einen Phenylrest, eine Cyangruppe oder ein Halogenatom substituiert sein kann,

bedeutet, auch zur Herstellung der erfindungsgemäßen Tetrahydropyrimidin-Verbindungen angewandt werden kann.

Die Synthese besteht in der Umsetzung von N-substituierten, zwei Propylendiamin-Einheiten enthaltenden Verbindungen der allgemeinen Formel V

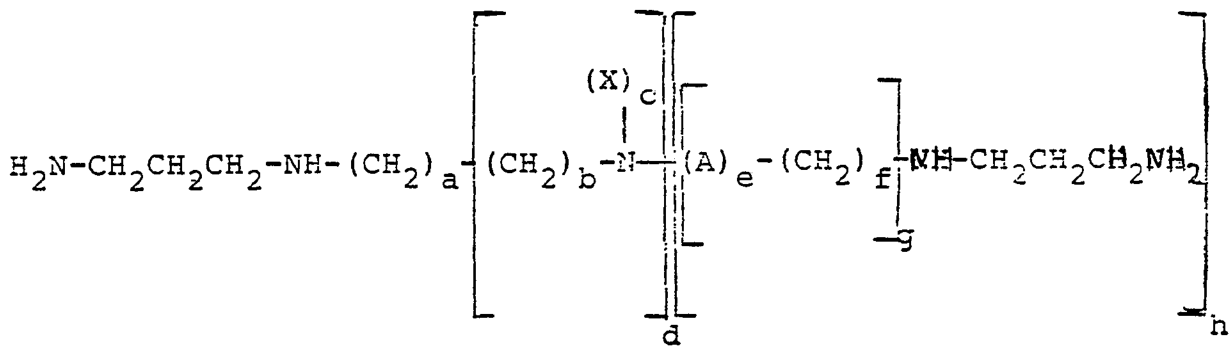

V

worin

A, X, a, b, c, d, e, f, g und h die oben angegebenen Bedeutungen haben, mit einem Acetessigsäurederivat der allgemeinen Formel VI

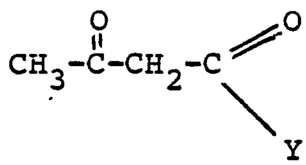

, VI

in der

Y für $-OR^4$, $-NHR^4$ oder $-NR^4_2$ und $R^4$ für einen $C_6$-$C_{10}$-Aryl-, $C_1$-$C_{17}$-Alkyl- oder $C_5$-$C_7$-Cycloalkylrest steht,

in Abwesenheit von Katalysatoren bei einer Reaktionstemperatur von 0-80°C umsetzt und dabei das bei der Reaktion entstehende Wasser kontinuierlich auskreist.

Die Synthese ergibt die gewünschten Produkte in im allgemeinen hoher Reinheit und hoher Ausbeute, die Reaktion ist regioselektiv. Dies ist insofern überraschend, da gegenüber dem in DE-AS 2 439 550 angegebenen Verfahren die Zahl der reaktiven Zentren vergrößert ist,

so daß dadurch auch die möglichen inter- und intramolekularen Reaktionen, die zu anderen als den gewünschten Produkten führen würden, an Wahrscheinlichkeit gewinnen.

Die in das erfindungsgemäße Verfahren einzusetzenden, an beiden Enden 1,3-Diaminopropaneinheiten besitzenden N-substituierten Amine sind allgemein solche, wie sie durch Umsetzung von Acrylnitril mit 1,ω-Diaminen und nachfolgende Reduktion entstehen. Solche Amine sind z.B.:
1,2-Diaminoethan, 1,4-Diaminobutan, 1,6-Diaminohexan, 1,12-Diaminododekan, 1,5-Diamino-3-oxapentan, 1,8-Diamino-3,6-dioxaoktan, 3-Methyl-3-aza-1,5-diaminopentan, 1,8-Diamino-3-Methyl-3-azaoktan, 1-Aminomethyl-5-amino-1,3,3-trimethylcyclohexan, 1,4-Diaminocyclohexan, 1,4-Diaminomethylbenzol, Hydrolyseprodukte von NCO-Prepolymeren, wie sie durch Umsetzung von aliphatischen Polyisocyanaten mit Polyhydroxyverbindungen im Molverhältnis NCO:OH = 2 entstehen, ferner die Verbindungen

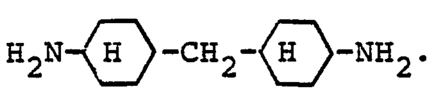

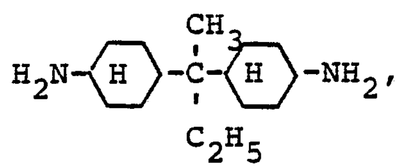

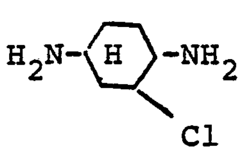

,

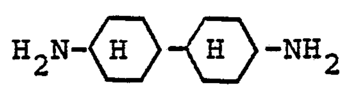

Als Acetessigsäurederivate kommen für das erfindungsgemäße Verfahren Acetessigsäureester und mono- oder di-substituierte Acetessigsäureamide in Frage. Innerhalb der allgemeinen Formel

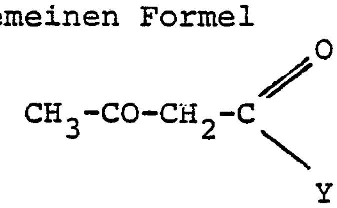

sind solche Acetessigsäurederivate bevorzugt, bei denen

Y für $-OR^4$, $-NHR^4$ oder $-NR^4_2$ steht und

$R^4$ einen $C_1$-$C_{17}$-Alkyl-, einen $C_5$-$C_7$-Cycloalkyl- oder einen $C_6$-$C_{10}$-Arylrest

bedeutet.

Ganz besonders bevorzugt sind Acetessigsäureester (Y = $OR^4$), in denen $R^4$ für einen gesättigten $C_1$-$C_6$-Alkyl- oder einen $C_5$-$C_6$-Cycloalkylrest steht.

Als Beispiele für einsetzbare Acetessigsäurederivate seien folgende Einzelverbindungen genannt: Acetessigsäuremethylester, Acetessigsäureethylester, Acetessigsäurebutylester, Acetessigsäurebenzylester, Acetessigsäurephenylester, Acetessigsäure-N-methylamid, Acetessigsäure-N-diethylamid, Acetessigsäure-N-butylamid, Acetessigsäure-N-dipropylamid, Acetessigsäure-N-benzylamid, Acetessigsäure-N-cyclohexylamid, Acetessigsäure-N-phenylamid.

Die in das erfindungsgemäße Verfahren einsetzbaren Acetessigsäurederivate sind in an sich bekannter Weise durch Umsetzung von Diketen mit den entsprechenden Alkoholen bzw. N-mono- oder N-disubstituierten Aminen leicht zugänglich.

Das erfindungsgemäße Verfahren wird im allgemeinen wie folgt durchgeführt: Entweder wird das eingesetzte N-substituierte Propylen-diamin vorgelegt und das

Acetessigsäurederivat zugegeben, oder das Acetessigsäurederivat wird vorgelegt und N-substituiertes Propylen-diamin zugegeben. Es ist möglich, in Anwesenheit oder Abwesenheit eines Lösungsmittels zu arbeiten. Die Reaktionstemperatur wird auf 0 - 80°C eingestellt. Das bei der Reaktion entstehende Wasser wird bei ebenfalls 0 - 80°C entfernt, wobei dies durch Zusatz eines gegenüber Ausgangs- und Reaktionsprodukten inerten Wasser entziehenden Mittels oder durch Destillation, gegebenenfalls unter vermindertem Druck, oder durch azeotrope Destillation, gegebenenfalls unter vermindertem Druck, erfolgen kann. Die Aufarbeitung des verbleibenden Reaktionsgemisches zur Gewinnung von 1-substituiertem 2-Methyl-tetrahydropyrimidin erfolgt in an sich bekannter Weise, beispielsweise durch Destillation oder Extraktion.

Die Ausgangsverbindungen werden zweckmäßigerweise in molarem Verhältnis eingesetzt. Es kann jedoch auch vorteilhaft sein, eine der Ausgangsverbindungen im Überschuß, beispielsweise in einem Überschuß bis zu 1,5 Mol pro Mol der zweiten Ausgangsverbindung einzusetzen.

Vorzugsweise wird in Gegenwart eines Lösungsmittels gearbeitet. Das Lösungsmittel soll zweckmäßigerweise gegenüber Ausgangs- und Reaktionsprodukten inert sein. Beispielsweise kommen Halogenkohlenwasserstoffe, aromatische Kohlenwasserstoffe, Ester und Ether als Lösungsmittel in Frage. Bevorzugt werden

solche inerten Lösungsmittel verwendet, mit denen auch anschließend das bei der Reaktion entstandene Wasser azeotrop abdestilliert werden kann. Dementsprechend können solche inerten Lösungsmittel mit Vorteil eingesetzt werden, die bei Normaldruck unter 150°C sieden und mit Wasser ein Azeotrop bilden. Derartige Lösungsmittel können beispielsweise Halogenkohlenwasserstoffe und aromatische Kohlenwasserstoffe sein. Beispielsweise seien Dichlormethan, Trichlormethan, Benzol, Toluol, Xylol und Chlorbenzol genannt.

Die Reaktionstemperatur beim erfindungsgemäßen Verfahren wird vorzugsweise auf 10 - 70°C, ganz besonders bevorzugt auf 20 - 50°C eingestellt. Die Entfernung des bei dem erfindungsgemäßen Verfahren entstehenden Wassers erfolgt bevorzugt bei 10 - 70°C, ganz besonders bevorzugt bei 20 - 50°C. Wie bereits erwähnt, kann die Wasserentfernung beispielsweise durch Destillation, azeotrope Destillation oder durch Zugabe eines inerten wasserentziehenden Mittels erfolgen.

Wird das Reaktionswasser durch Destillation entfernt, so wird diese Destillation vorzugsweise im Vakuum durchgeführt, damit bei der Wasserentfernung die Temperaturen unter 80°C gehalten werden können. Beispielsweise können Drücke von 10 - 700 Torr angewendet werden.

Sofern das Reaktionswasser durch Zugabe eines inerten wasserentziehenden Mittels entfernt werden

soll, wird dieses zweckmäßigerweise mindestens in einer solchen Menge zugesetzt, daß die theoretisch berechnete Menge entstehenden Wassers gebunden werden kann. Vorteilhaft wird ein Überschuß an wasserentziehendem Mittel eingesetzt. Geeignete wasserentziehende Mittel sind beispielsweise wasserfreie Soda, Zeolithe, Bariumoxid und Calciumoxid.

Vorzugsweise erfolgt die Wasserentfernung durch azeotrope Destillation unter Verwendung eines der bereits beschriebenen azeotropbildenden Lösungsmittel. Wird die azeotrope Destillation diskontinuierlich durchgeführt, so wird das azeotropbildende Lösungsmittel zweckmäßigerweise mindestens in einer solchen Menge zugesetzt, daß die theoretisch berechnete Menge entstehenden Reaktionswassers entfernt werden kann. Vorzugsweise wird ein Überschuß an azeotropbildendem Lösungsmittel verwendet. Bei kontinuierlicher Durchführung der azeotropen Destillation kann auch weniger azeotropbildendes Lösungsmittel eingesetzt werden. Hierbei wird nach Abkühlung und Phasentrennung des abdestillierten Azeotrops die wässrige Phase entfernt und das azeotropbildende Lösungsmittel zurückgeführt. Dieser Kreislauf wird dann solange aufrechterhalten, bis aus dem Reaktionsgemisch kein Wasser mehr ausgetragen wird. Die während der Entfernung des Reaktionswassers durch azeotrope Destillation einzuhaltenden Druckbedingungen richten sich nach dem sich bildenden Azeotrop. Der Druck wird so gewählt, daß die Temperatur (im Reaktionsgemisch gemessen) bei

der azeotropen Destillation nicht über 80°C ansteigt. Sofern sich Azeotrope bilden, die bei Normaldruck unter 80°C sieden, kann bei Normaldruck oder vermindertem Druck gearbeitet werden. Geeignete Drücke sind in diesem Fall beispielsweise solche von 10 Torr bis Normaldruck, insbesondere solche von 15 - 50 Torr. Bilden sich Azeotrope mit einem bei Normaldruck höheren Siedepunkt als 80°C, muß bei vermindertem Druck gearbeitet werden. Geeignete Drücke sind in diesem Fall beispielsweise solche von 10 - 200 Torr, insbesondere solche von 15 - 50 Torr.

Die Gewinnung des Tetrahydropyrimidins aus dem nun vorliegenden Gemisch erfolgt vorzugsweise durch Destillation. Der Druck, bei dem diese Destillation durchgeführt wird, kann in weiten Grenzen variiert werden, da nunmehr auch Temperaturen von über 80°C auftreten können, ohne daß in nennenswertem Umfang Nebenreaktionen ablaufen. Beispielsweise können solche Drücke angewendet werden, daß das abzutrennende Tetrahydropyrimidin zwischen 0 und 300°C siedet. Vorzugsweise wird bei Normaldruck oder vermindertem Druck, beispielsweise bei 0,01 - 760 Torr gearbeitet. Besonders bevorzugt sind Drücke von etwa 0,1 - 50 Torr und eine Siedetemperatur des abzutrennenden Tetrahydropyrimidins im Bereich von 100 - 300°C.

Während das Verfahren gemäß Chem. Ber. 98, 3652 (1958) bei hoher Temperatur und in Gegenwart von Toluolsulfonsäure als Katalysator durchgeführt wird,

0054876

wobei das Reaktionswasser relativ lange im Reaktionsgemisch verbleibt, liegt dem erfindungsgemäßen Verfahren die grundlegende Erfindung zugrunde, daß hohe Ausbeuten an 1-substituierten 2-Methyltetrahydropyrimidinen nur dann erhalten werden, wenn bei tiefen Temperaturen und ohne Katalysator gearbeitet wird. Darüber hinaus wurde gefunden, daß das Reaktionswasser bei tiefer Temperatur dem Reaktionsansatz entzogen werden muß, um eine Hydrolyse des cyclischen Amidins zu verhindern.

Mit dem erfindungsgemäßen Verfahren können die Tetrahydropyrimidine auch in technischem Maßstab einfach und in guter Ausbeute hergestellt werden. Das erfindungsgemäße Verfahren kann auch ohne Schwierigkeiten kontinuierlich durchgeführt werden.

Typische Protenendonatorverbindungen bzw. Protonensäuren für die Herstellung von Additionsverbindungen, Salzen und Komplexen mit den erfindungsgemäßen Tetrahydropyrimidin-Verbindungen sind z.B. Monocarbonsäuren, wie Ameisensäure, Essigsäure, Propionsäure, Buttersäure, Capronsäure, Valeriansäure, Octylsäure, Laurinsäure, Stearinsäure und Ölsäure, Dicarbonsäuren wie Oxalsäure, Malonsäure, Bernsteinsäure, Fumarsäure und Adipinsäure, Hydroxysäuren, wie Glykolsäure, Milchsäure und Weinsäure, Sulfonsäuren, wie Alkyl- oder Aryl-Sulfonsäuren, Sulfaminsäure und Sulfanilsäure. Anorganische Säuren, wie Kohlensäure, Phosphorsäure, Salzsäure und Schwefelsäure und

andere Protonendonator-Verbindungen, wie Sulfonamide, Phenole, wie Phenol und Kresol, Enole, wie Barbitursäure und Harnsäure. Besonders bevorzugt sind Fettsäuren mit wenigstens zwei Kohlenstoffatomen, Phenole und Kohlensäure.

Zur Herstellung der "Säureadditionssalze" setzt man einfach die freie Base mit einer stöchiometrischen Menge der ausgewählten Protonendonator-Verbindungen um. Die Salzbildung tritt leicht bei Raumtemperatur ohne Hilfe eines Katalysators ein.

Wenn feste Reaktionsteilnehmer verwendet werden, kann es vorteilhaft sein, ein inertes, flüssiges Lösungsmittel, wie Benzol, Toluol, Xylol, Hexan, Heptan, Dichlormethan oder Trichlormethan als Reaktionsmedium zu verwenden. Das Lösungsmittel kann dann am Abschluß der Umsetzung leicht nach herkömmlichen Verfahren entfernt werden. Irgendeine weitere Reinigung des "Säureadditionssalzes" ist nicht erforderlich, wenn man auch eine Umkristallisation aus einem Lösungsmittel, wie Isooctan, anwenden kann, falls ein Produkt von hoher Reinheit gewünscht wird.

Lewissäuren sind bekanntlich im weiten Sinne Elektronenmangelverbindungen. Dieser Begriff wird z.B. in dem Lehrbuch "Anorganische Chemie" von F.A. Cotton und G. Wilkinson, 2. Auflage, Verlag Chemie GmbH, Weinheim/Bergstraße 1968 oder in dem Lehrbuch von Edwin S. Gould "Mechanismus und Struktur in der

Organischen Chemie", 2. Auflage, Verlag Chemie GmbH, Weinheim/Bergstr. 1969 erläutert, dort sind auch Beispiele für Lewissäuren angegeben.

Einige Beispiele für Lewissäuren sind: $SO_2$, $SO_3$, $BF_3$, $BCl_3$, $BI_3$, $Al_2Cl_6$, $SnCl_4$.

Erfindungsgemäß sind folgende Tetrahydropyrimidine bevorzugt:

1,2-Bis(2-methyl-tetrahydropyrimidinoyl-1)ethan der Formel

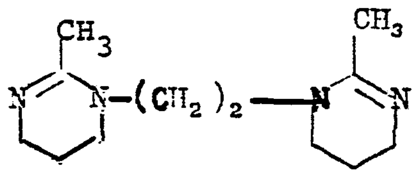

1,4-Bis(2-methyl-tetrahydropyrimidinoyl-1)butan der Formel

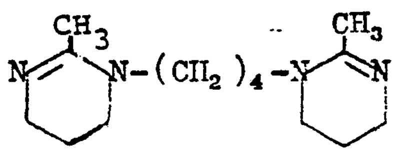

1,6-Bis(2-methyl-tetrahydropyrimidinoyl-1)hexan der Formel

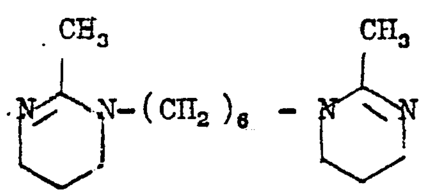

1,5-Bis(2-methyl-tetrahydropyrimidinoyl-1)-3-oxapentan der Formel

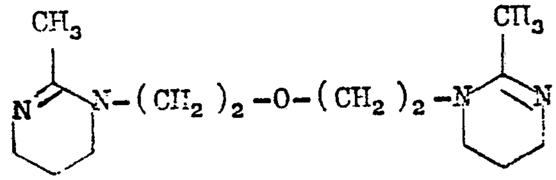

1,8-Bis(2-methyl-tetrahydropyrimidinoyl-1)-3,6-dioxaoctan der Formel

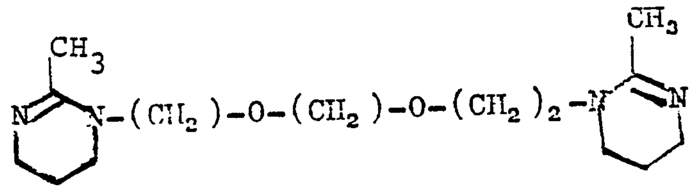

1,9-Bis(2-methyl-tetrahydropyrimidinoyl-1)-5-oxanonan der Formel

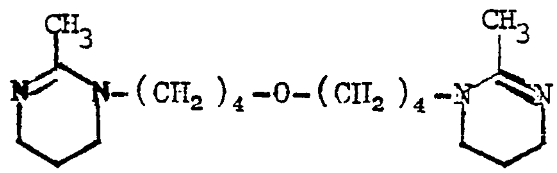

1,5-Bis(2-Methyl-tetrahydropyrimidinoyl-1)-3-methyl-3-azapentan der Formel

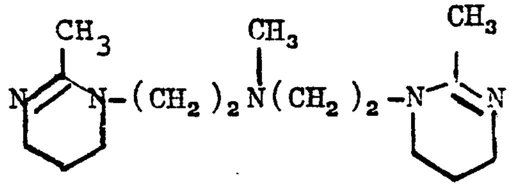

1,5-Bis(2-methyl-tetrahydropyrimidinoyl-1)-3-n-dodecyl-3-azapentan der Formel

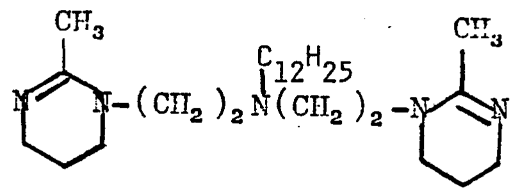

1,8-Bis(2-methyl-tetrahydropyrimidinoyl-1)-3,6-dimethyl-3,6-diazaoctan der Formel

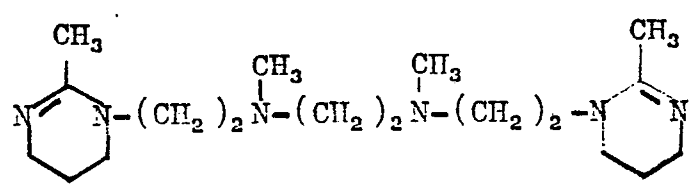

0054876

1,7-Bis(2-methyl-tetrahydropyrimidinoyl-1)-4-methyl-4-azaheptan der Formel

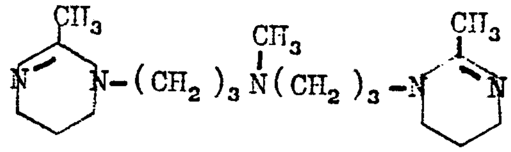

1,23-Bis(2-methyl-tetrahydropyrimidinoyl-1)-10,14-dimethyl-8,16-dioxo-7,17-diaza-9,12,15-trioxa-tricosan der Formel (idealisiert)

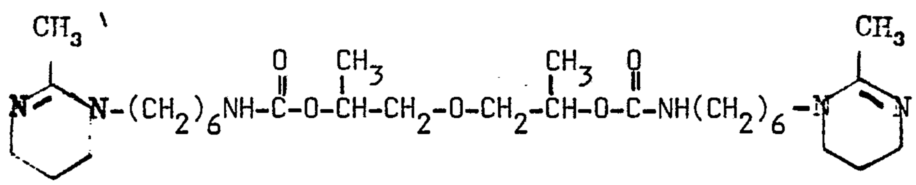

1-(3-Di-N-methylaminopropyl)-2-methyl-tetrahydropyrimidin der Formel

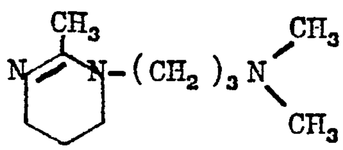

sowie deren Salze, Additionsverbindungen und Komplexe mit Proton- und Lewissäuren.

Besonders bevorzugt sind

1,5-Bis(2-methyl-tetrahydropyrimidinoyl-1)-3-oxapentan und
1,7-Bis(2-methyl-tetrahydropyrimidinoyl-1)-4-methyl-4-azaheptan.

Die erfindungsgemäßen Tetrahydropyrimidine stellen niederschmelzende oder flüssige Verbindungen dar,

die sich im allgemeinen gut in den zu Herstellung von Polyurethanschaumstoffen eingesetzten, hoch- oder niedermolekularen Polyhydroxylverbindungen lösen. Es ist jedoch auch möglich, die Tetrahydropyrimidin-Komponente in fein dispergierter Form einzusetzen.

Die erfindungsgemäßen, Tetrahydropyrimidin-Einheiten enthaltenden Katalysatoren sind geruchlos, bzw. haben einen geringen, angenehmen Geruch. Sie besitzen nicht den typischen, unangenehmen "Amingeruch".

Erfindungsgemäß werden die neuen Tetrahydropyrimidine als Katalysatoren bei der Herstellung von Polyurethankunststoffen verwendet. Als Ausgangsmaterialien für die Herstellung der Polyurethankunststoffe kommen organische Polyisocyanate in Frage, wie sie in der DE-OS 28 54 384, Seite 8, Zeile 13 -Seite 11, Zeile 20 beschrieben werden. Reaktionspartner für diese Polyisocyanate sind Verbindungen mit mindestens zwei gegenüber Isocyanaten reaktionsfähigen Wasserstoffatomen von einem Molekulargewicht von 400 - 10000, wie sie in der DE-OS 28 54 384, Seite 11, Zeile 21 - Seite 19, Zeile 29 ausführlich offenbart werden. Bei der Herstellung der Polyurethankunststoffe werden gegebenenfalls Verbindungen mit mindestens zwei gegenüber Isocyanaten reaktionsfähigen Wasserstoffatomen und einem Molekulargewicht von 32 - 400 als Kettenverlängerungsmittel bzw. Vernetzungsmittel mitverwendet. Derartige Verbindungen werden z.B. in der DE-OS 28 54 384, Seite 20, Zeile 1 - Seite 26, Zeile 5

beschrieben. Ferner können gegebenenfalls Treibmittel und weitere Hilfs- und Zusatzstoffe miteingesetzt werden; die in Frage kommenden Treibmittel bzw. Hilfs- und Zusatzstoffe werden in der DE-OS 28 54 384, Seite 26, Zeile 6 - Seite 31, Zeile 17 beschrieben.

Die erfindungsgemäßen Katalysatoren werden in Mengen von 0,01-10 Gew.-%, bevorzugt 0,05-5 Gew.-%, besonders bevorzugt 0,1-2 Gew.-%, bezogen auf die eingesetzte Menge an Verbindungen mit mindestens zwei aktiven Wasserstoffatomen vom Molekulargewicht 400-10000, verwendet.

Bevorzugt werden die erfindungsgemäßen Tetrahydropyrimidin-Katalysatoren in Kombination mit anderen aus der Polyurethan-Chemie bekannten Katalysatoren, bevorzugt aminischen und Metallkatalysatoren eingesetzt, wobei die verwendete Gesamt-Katalysatormenge zu mindestens 2 Gew.-%, bevorzugt zu mindestens 20 Gew.-%, aus den erfindungsgemäßen Tetrahydropyrimidin-Katalysatoren besteht.

Solche zusätzlich zu den erfindungsgemäßen Katalysatoren zu verwendenden Katalysatoren sind:
Tertiäre Amine, wie Triethylamin, Tributylamin, N-Methyl-morpholin, N-Ethyl-morpholin, N,N,N',N'-Tetramethyl-ethylendiamin, Pentamethyl-diethylentriamin und höhere Homologe (DE-Offenlegungsschriften 2 624 527 und 2 624 528), 1,4-Diazabicyclo-(2,2,2)-octan, N-Methyl-N'-dimethylaminoethyl-

piperazin, Bis(dimethylaminoalkyl)-piperazine (DE-Offenlegungsschrift 2 636 787), N,N-Dimethylbenzylamin, N,N-Dimethylcyclohexylamin, N,N-Diethylbenzylamin, Bis-(N,N-diethylaminoethyl)-adipat, N,N,N',N'-Tetramethyl-ethylendiamin, Pentamethyl-diethylentriamin und höhere Homologe (DE-Offenlegungsschriften 2 624 527 und 2 624 528), 1,4-Diaza-bicyclo-(2,2,2)-octan, N-Methyl-N'-dimethylaminoethylpiperazin, Bis(dimethylaminoalkyl)-piperazine (DE-Offenlegungsschrift 2 636 787), N,N-Dimethylbenzylamin, N,N-Dimethylcyclohexylamin, N,N-Diethylbenzylamin, N,N,N',N'-Tetramethyl-1,3-butandiamin, N,N-Dimethyl-ß-phenylethylamin, 1,2-Dimethylimidazol, 2-Methylimidazol, monocyclische und bicyclische Amidine (DE-Offenlegungsschrift 1 720 633), Bis(dialkylamino)alkyl-ether (US-Patentschrift 3 330 782, DE-Auslegeschrift 1 030 558, DE-Offenlegungsschriften 1 804 361 und 2 618 280) sowie Amidgruppen (vorzugsweise Formamidgruppen) aufweisende tertiäre Amine gemäß den DE-Offenlegungsschriften 2 523 633 und 2 732 292). Als zusätzliche Katalysatoren kommen auch an sich bekannte Mannichbasen aus sekundären Aminen, wie Dimethylamin, und Aldehyden, vorzugsweise Formaldehyd, oder Ketonen wie Aceton, Methylethylketon oder Cyclohexanon und Phenolen, wie Phenol, Nonylphenol oder Bisphenol, in Frage.

Gegenüber Isocyanatgruppen aktive Wasserstoffatome aufweisende tertiäre Amine als zusätzlicher Katalysator sind z.B. Triethanolamin, Triisopropanolamin, N-Methyl-diethanolamin, N-Ethyl-diethanolamin, N,N-Dimethyl-ethanolamin, deren Umsetzungsprodukte mit Alkylenoxiden wie Propylenoxid und/oder Ethylenoxid sowie sekundär-tertiäre Amine gemäß DE-Offenlegungsschrift 2 732 292.

Als zusätzliche Katalysatoren kommen ferner Silaamine mit Kohlenstoff-Silizium-Bindungen, wie sie z.B. in der DE-Patentschrift 1 229 290 (entsprechend der US-Patentschrift 3 620 984) beschrieben sind, in Frage, z.B. 2,2,4-Trimethyl-2-silamorpholin und 1,3-Diethylaminomethyl-tetramethyl-disiloxan.

Als zusätzliche Katalysatoren kommen auch stickstoffhaltige Basen wie Tetraalkylammoniumhydroxide, ferner Alkalihydroxide wie Natriumhydroxid, Alkaliphenolate wie Natriumphenolat oder Alkalialkoholate wie Natriummethylat in Betracht. Auch Hexahydrotriazine können als zusätzliche Katalysatoren eingesetzt werden (DE-Offenlegungsschrift 1 769 043).

Die Reaktion zwischen NCO-Gruppen und Zerewitinoffaktiven Wasserstoffatomen wird auch durch Lactame und Azalactame stark beschleunigt, wobei sich zunächst ein Assoziat zwischen dem Lactam und der Verbindung mit acidem Wasserstoff ausbildet. Derartige Assoziate und ihre katalytische Wirkung werden in den DE-Offenlegungsschriften 2 062 288, 2 062 289, 2 117 576 (US-Patentschrift 3 758 444), 2 129 198, 2 330 175 und 2 330 211 beschrieben.

Erfindungsgemäß können auch organische Metallverbindungen, insbesondere organische Zinnverbindungen, organische Blei- und organische Wismutverbindungen als zusätzliche Katalysatoren, verwendet werden. Als organische Zinnverbindungen kommen neben schwefelhaltigen Verbindungen wie Di-n-octyl-zinn-mercaptid (DE-Auslegeschrift 1 769 367; US-Patentschrift 3 645 927) vorzugsweise Zinn(II)-salze von Carbonsäuren wie Zinn(II)-acetat, Zinn(II)-octoat, Zinn(II)-ethylhexoat und Zinn(II)-laurat und die Zinn(IV)-Verbindungen, z.B. Dibutylzinnoxid, Dibutylzinndichlorid, Dibutylzinndiacetat, Dibutylzinndilaurat, Dibutylzinnmaleat oder Dioctylzinndiacetat, Bleiverbindungen wie Pb (II)-oktoat und Wismutverbindungen wie Bi(III)oktoat oder Bi(III)thiododecylat in Betracht.

Selbstverständlich können alle obengenannten Katalysatoren als Gemische eingesetzt werden. Von besonderem Interesse sind dabei Kombinationen aus organischen Metallverbindungen und Amidinen, Aminopyridinen oder Hydrazinopyridinen (DE-Offenlegungsschriften 2 434 185, 2 601 082 und 2 603 834).

Die zusätzlichen Katalysatoren werden in der Regel in einer Menge zwischen etwa 0,001 und 10 Gew.-%, bezogen auf die Gesamtmenge an Verbindungen mit mindestens zwei gegenüber Isocyanaten reaktionsfähigen Wasserstoffatomen vom Molekulargewicht 400 - 10.000 und in Mengen von 0-98 Gew.-%, bevorzugt zu 0-80 Gew.-%, bezogen auf die Gesamtmenge der verwendeten Katalysatoren, eingesetzt.

Für die erfindungsgemäßen Salze, Additionsverbindungen und Komplexe der Tetrahydropyrimidine gilt sinngemäß gleiches. Bei der Verwendung dieser Salze, Additionsverbindungen und Komplexe in Kombination mit Co-Katalysatoren, wie sie oben erwähnt wurden, muß jedoch darauf geachtet werden, daß keine Austauschreaktionen etc. zwischen den Katalysatorkomponenten auftreten, die den beabsichtigten Effekt zunichte machen können.

A. Eine besondere Ausführungsform der Polyurethankunststoff-Herstellung gemäß Erfindung besteht darin, daß als Katalysatoren Carbonate und Hydrogencarbonate der neuen Tetrahydropyrimidine ggf. im Gemisch mit anderen Hydrogencarbonaten und Carbonaten von bekannten Amin- und Amidin-Katalysatoren, eingesetzt werden und daß dann

B. keine weiteren physikalischen Treibmittel verwendet werden. Auf diese Weise wirken die neuen Tetrahydropyrimidin-Salze sowohl als Katalysatoren als auch als Treibmittel.

Durchführung der Polyurethankunststoff-Herstellung:

Die Reaktionskomponenten werden erfindungsgemäß nach dem an sich bekannten Einstufenverfahren, dem Prepolymerverfahren oder dem Semiprepolymerverfahren zur Umsetzung gebracht, wobei man sich oft maschineller Einrichtungen bedient, z.B. solcher, die in der US-Patentschrift 2 764 565 beschrieben werden. Einzelheiten über Verarbeitungseinrichtungen, die

auch erfindungsgemäß in Frage kommen, werden im Kunststoff-Handbuch, Band VII, herausgegeben von Vieweg und Höchtlen, Carl-Hanser-Verlag, München 1966, z.B. auf den Seiten 121 bis 205 beschrieben.

Bei der Schaumstoffherstellung kann erfindungsgemäß die Verschäumung auch in geschlossenen Formen durchgeführt werden. Dabei wird das Reaktionsgemisch in eine Form eingetragen. Als Formmaterial kommt Metall, z.B. Aluminium, oder Kunststoff, z.B. Epoxidharz, in Frage. In der Form schäumt das schäumfähige Reaktionsgemisch auf und bildet den Formkörper. Die Formverschäumung kann dabei so durchgeführt werden, daß das Formteil an seiner Oberfläche Zellstruktur aufweist, sie kann aber auch so durchgeführt werden, daß das Formteil eine kompakte Haut und einen zelligen Kern aufweist. Erfindungsgemäß kann man in diesem Zusammenhang so vorgehen, daß man in die Form so viel schäumfähiges Reaktionsgemisch einträgt, daß der gebildete Schaumstoff die Form gerade ausfüllt. Man kann aber auch so arbeiten, daß man mehr schäumfähiges Reaktionsgemisch in die Form einträgt, als zur Ausfüllung des Forminneren mit Schaumstoff notwendig ist. Im letztgenannten Fall wird somit unter "overcharging" gearbeitet; eine derartige Verfahrensweise ist z.B. aus den US-Patentschriften 3 178 490 und 3 182 104 bekannt.

Bei der Formverschäumung werden vielfach an sich bekannte "äußere Trennmittel", wie Siliconöle, mitverwendet. Man kann aber auch sogeannte "innere Trennmittel", gegebenenfalls im Gemisch mit äußeren Trennmitteln, verwenden, wie sie z.B. aus den DE-Offenlegungsschriften 2 121 670 und 2 307 589 bekanntgeworden sind.

Erfindungsgemäß lassen sich auch kalthärtende Schaumstoffe herstellen (vgl. GB-Patentschrift 1 162 517, DE-Offenlegungsschrift 2 153 086).

Selbstverständlich können aber auch Schaumstoffe durch Blockverschäumung oder nach dem an sich bekannten Doppeltransportbandverfahren hergestellt werden.

Die nach der Erfindung erhältlichen Produkte finden z.B. folgende Anwendung: Als Schuhsohlen, Polstermaterial, Schalldämmstoffe, Beschichtungsmittel, Lacke, Verpackungsmaterial, Matratzen.

## Beispiele

Beispiel 1: Herstellung von 1,2-Bis(2-methyl-tetrahydropyrimidinoyl-1)ethan

174 g (1 Mol) 1,10-Diamino-4,7-diazadecan, 260 g (2 Mol) Acetessigester und 250 ml Toluol werden zusammengegeben und 2 h lang bei einer Kolbeninnentemperatur von 40°C und einem Druck von 20 Torr gehalten. Dabei werden in einem evakuierbaren Wasserabscheider 36 g Wasser (100 %) abgeschieden. Nach Beendigung der azeotropen Vakuumdestillation wird bei Normaldruck und einer Kolbeninnentemperatur von 160°C zuerst Toluol und dann Essigester (111 g nach 4 h, entspricht 1.26 Mol) destilliert. Die destillative Aufarbeitung des erstarrten Rückstands erfolgt im Hochvakuum, bei 0.03 Torr gehen bei 187-193°C 185 g (83.3 % d.Th.) eines farblosen Produktes über, das in der Vorlage erstarrt und dann bei 121-123°C schmilzt. Die gaschromatographische Analyse weist das Produkt als 97-98%ig rein aus.

Analysendaten: $C_{12}H_{22}N_4$ (Molekulargewicht: 222)

| | | | |
|---|---|---|---|
| Ber.: | C 64.9 | H 10.0 | N 25.2 |
| Gef.: | C 64.3 | H 9.8 | N 25.4 |

Beispiel 2: Herstellung von 1,6-Bis(2-methyltetrahydropyrimidinoyl-1)hexan

147 g 1,14-Diamino-4,11-diazatetradecan (0.64 Mol), 300 ml Toluol und 166,6 g Acetessigester (1,28 Mol)

werden zusammengegeben und 2 h bei einem Druck von 20 Torr auf maximal 40°C erwärmt. Dabei gehen 23 g Wasser (100 %) über, die mittels eines evakuierbaren Wasserabscheiders abgetrennt werden. Zur Ausscheidung des Solvens und von Essigester wird 4 h auf 160°C erhitzt. Der Rückstand wird im Hochvakuum destilliert, bei 0,1 Torr und einer Übergangstemperatur von 225 - 245°C gehen 161 g (90 %) eines gelblichen Produktes über, das laut gaschromatographischer Analyse zu 96-97 % rein ist.

Analysendaten: $C_{16}H_{30}N_4$ (Molekulargewicht 278)

| | | | |
|---|---|---|---|
| Ber.: | C 69.1 | H 10.8 | N 20.1 |
| Gef.: | C 68.4 | H 10.8 | N 19.7 |

$n_D^{23}$ : 1.5220

Beispiel 3: Herstellung von 1,7-Bis(2-methyl-tetrahydropyrimidinoyl-1)-4-methyl-4-azaheptan

750 g 1,15-Diamino-8-methyl-4,8,12-triazatetradecan (2,9 Mol), 753 g Acetessigester (5,8 Mol) und 800 ml Toluol werden zusammengegeben und 3 h bei einem Druck von 20 Torr auf maximal 40°C erwärmt. Dabei gehen 105 g Wasser (100 %) über, die mittels eines evakuierbaren Wasserabscheiders aus dem Reaktionsgemisch abgetrennt werden. Toluol und sich abspaltender Essigester werden bei Normaldruck und 140°C Badtemperatur abdestilliert. Der Rückstand, der lt. $^1$H-NMR zu 90 %

0054876

rein ist, wird im Hochvakuum destilliert. Bei 0,1 Torr, einer Badtemperatur von 260-280°C gehen bei 220-240°C 736 g (83 %) eines gelbgefärbten Produkts über, das ein korrektes $^{1}$H-NMR-Spektrum zeigt und folgende Analysendaten aufweist:

$C_{17}H_{33}N_5$ (Molekulargewicht 307)

| | | | | |
|---|---|---|---|---|
| Ber. | : | C 66.5 | H 10.8 | N 22.8 |
| Gef. | : | C 66.1 | H 11.3 | N 22.3 |
| $n_D^{23}$ | : | 1.5228 | | |

Beispiel 4: Herstellung von 1,23-Bis(2-methyl-tetrahydropyrimidinoyl-1)-10,14-dimethyl-8,16-dioxo-9,12,15-trioxa-7,17-diaza-tricosan(idealisiert)

4.1.

Als Ausgangsamin wird ein Polyamin verwendet, wie es durch Umsetzung von Acrylnitril und nachfolgender Hydrierung mit einem Diamin erhalten wurde, das hergestellt wurde durch Hydrolyse eines NCO-Präpolymeren aus 1,6-Diisocyanatohexan und techn. Dipropylenglykol und eine NH-Zahl von 205 hat.

4.2.

197 g 1,31-Diamino-4,11,21,28-tetrazo-14,18-dimethyl-12,20-dioxo-13,16,19-trioxa-pentriacontan(0,372 Mol), dessen Darstellung unter 4.1. skizziert wurde, wird mit 96.7 g (0,744 Mol) Acetessigester und 500 ml Toluol vermischt und bei 20 Torr 2 h auf 40°C erwärmt. Dabei gehen 14 ml Wasser (100 %) über, die mittels eines evakuierbaren Wasserabscheiders aus dem Gleichgewicht entfernt werden. Toluol und Essigester werden bei 140°C bei Normaldruck und anschließend bei 0,1 Torr/100°C abdestilliert. Es hinterbleibt ein von bei ca. 80°C schmelzenden Kristallen durchsetzter breiartiger Rückstand, dessen Analysendaten folgendermaßen lauten:

$C_{30}H_{56}N_6O_5$ (Molekulargewicht 590)

| | | | |
|---|---|---|---|
| Ber. : | C 61,0 | H 9,5 | N 14,2 |
| Gef.: | C 60,6 | H 9,5 | N 13,7 |

<u>Beispiel 5:</u> Herstellung von 1-(3-Di-N-methylaminopropyl)-2-methyl-tetrahydropyrimidin

532 g N,N-Dimethyl-1,7-diamino-4-azaheptan (3,3 Mol), 429 g Acetessigester (3,3 Mol) und 800 ml Toluol werden 2 h bei 20 Torr auf 40°C erwärmt, wobei 58,4 g Wasser (100 %) abgeschieden werden. Toluol und anfallender Essigester werden bei Normaldruck abdestilliert, Destillation im Vakuum (0,02 Torr) ergibt 490 g (81 %) eines farblosen Produkts mit einem Siedepunkt von 103°C/0,02 Torr, korrektem $^1H$-NMR-Spektrum und folgenden Analysendaten:

$C_9H_{19}N_3$ (Molekulargewicht 183)

| | | | |
|---|---|---|---|
| Ber. : | C 59,0 | H 10,4 | N 23,0 |
| Gef. : | C 59,7 | H 10,5 | N 22,6 |
| $n_D^{23}$ : | 1.4906 | | |

Beispiel 6: Herstellung von 1,2-Bis(2-methyl-tetrahydropyrimidinoyl-1)ethan-bis(hydrogencarbonat)

22,2 g 1,2-Bis(2-methyl-tetrahydropyrimidinoyl-1)ethan (0,1 Mol), 3,6 g Wasser (0,2 Mol) werden in 300 ml Methanol gelöst und $CO_2$ bei 0° - 10°C bis zur Sättigung eingeleitet. Die ausgefallenen, abgesaugten und getrockneten Kristalle schmelzen bei 138-140°C. Ausbeute: 31,6 g ( 91 % der Theorie). Die analytischen Daten lauten:

$C_{18}H_{34}N_4O_6$ (Molekulargewicht 346)

| | | | |
|---|---|---|---|
| Ber. : | C 48,5 | H 7,5 | N 15,6 |
| Gef. : | C 49,0 | H 8,0 | N 15,8 |

<u>Beispiel 7:</u> Herstellung von 1,2-Bis(2-methyl-tetrahydropyrimidinoyl-1)ethan-bis(phenolat)

11,1 g 1,2-Bis(2-methyl-tetrahydropyrimidinoyl-1)ethan (50 mmol), 9,4 g Phenol (0,1 Mol) werden in 150 ml Aceton gelöst. Nach 2-stündigem Rühren wird das Aceton abdestilliert. Es hinterbleiben 20,5 g (100 %) einer hochviskosen Flüssigkeit, die nach einigen Tagen in bei 25-30°C schmelzenden Kristallen erstarrt.

Beispiele für die Herstellung von Polyurethankunststoffen

Ausgangsverbindungen

Isocyanate:

Isocyanat A: Semiprepolymer aus 4,4'-Diisocyanatodiphenylmethan und Tripropylenglykol mit einem NCO-Wert von 22,8 %.

Isocyanat B: Semiprepolymer aus Isophorondiisocyanat und einem auf Glyzerin gestarteten Polypropylenglykolether mit einer OH-Zahl von 670 mit einem NCO-Wert von 28 %.

Isocyanat C: Gemisch aus 80 Gew.-Teilen 2,4-Toluylendiisocyanat und 20 Gew.-Teilen 2,6-Toluylendiisocyanat mit einem NCO-Wert von 48 %.

Isocyanat D: Phosgenierungsprodukt von Anilin-Formaldehyd-Kondensaten, das eine Viskosität von 130mPas bei 25°C und einem NCO-Wert von 31 % aufweist.

Polyole:

Polyol A: Trifunktionelles, auf Trimethylolpropan gestartetes PO/EO-Polyetherpolyol (22 Gew.-% EO) mit einer OH-Zahl von 27 und einem mittleren Molekulargewicht von 5600.

Polyol B: Difunktionelles, auf Propylenglykol gestartetes PO/EO-Polyetherpolyol (20 Gew.-% EO) mit einer OH-Zahl von 28 und einem mittleren Molekulargewicht von 4000.

Polyol C: Trifunktionelles, auf Trimethylolpropan gestartetes PO/EO-Polyetherpolyol (15 Gew.-% EO) mit einer OH-Zahl von 35 und einem mittleren Molekulargewicht von 4500.

Polyol D: Polyolgemisch der mittleren OH-Zahl 500 und eines Wassergehaltes kleiner als 0,3 Gew.-% und einer Viskosität bei 25°C von 2500 mPas, bestehend aus

1) 60 Gew.-% eines Polyethers der OH-Zahl 860, der durch Anlagerung von Propylenglykol an Trimethylolpropan erhalten wurde, und

2) 40 Gew.-% eines Polyethers der OH-Zahl 42, der durch Anlagerung eines Gemisches von Propylenoxid und Ethylenoxid an ein Gemisch aus Trimethylolpropan und Propylenglykol (Molverhältnis 3:1) erhalten wurde.

In den Beispielen werden folgende Tetrahydropyrimidin-Katalysatoren verwendet.

Katalysator A: 1,2-Bis(2-methyl-tetrahydropyrimidinoyl-1)ethan der Formel

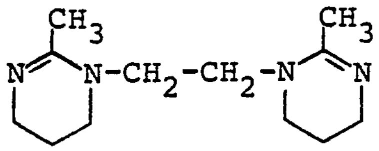

aus Beispiel 1.

Katalysator B: 1,2-Bis(2-methyl-tetrahydropyrimidinoyl-1)-ethan-bis(hydrogencarbonat) aus Beispiel 6

Katalysator C: 1,2-Bis(2-methyl-tetrahydropyrimidinoyl-1)-ethan-bis(phenolat) aus Beispiel 7.

Katalysator D: 1,6-Bis(2-methyl-tetrahydropyrimidinoyl-1)-hexan der Formel

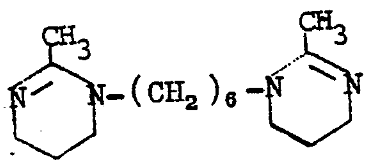

aus Beispiel 2.

Katalysator E: 1,7-Bis(2-methyl-tetrahydropyrimidinoyl-1)-4-methyl-4-azaheptan der allgemeinen Formel

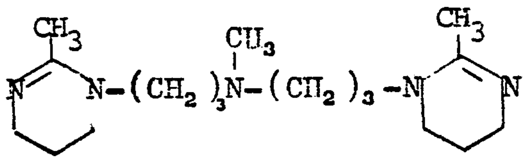

aus Beispiel 3.

Katalysator F: 1,23-Bis (2-methyl-tetrahydropyrimidinoyl-1)-10,14-dimethyl-8,16-dioxo-7,17-diaza-9,12,15-trioxatricosan der idealisierten Formel

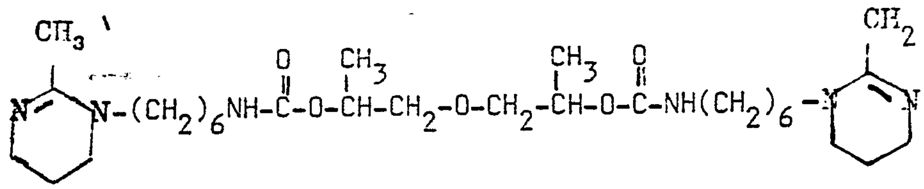

aus Beispiel 4.

Katalysator G: 1-(3-Di-N-methylaminopropyl)-2-methyl-tetrahydropyrimidin der Formel

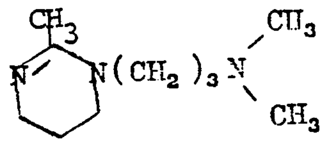

aus Beispiel 5.

Katalysator H: 1,2-Bis(2-methyl-tetrahydropyrimidinoyl-1)-ethan-bis(2-ethylhexanoat),

Katalysator I: 1,2-Bis(2-methyl-tetrahydropyrimidinoyl-1)-ethan-bis(acetat).

Die aufgeführten Reaktionszeiten sind folgendermaßen definiert:

$T_1$ = Startzeit (Zeit in Sekunden nach der Einrührung der Isocyanatkomponente, bei der das Gemisch zu schäumen beginnt);

$T_2$ = Steigzeit (Zeit in Sekunden nach der Einrührung der Isocyanatkomponente, nach der der Steigvorgang beendet ist);

$T_3$ = Klebfreizeit (Zeit in Sekunden nach der Einrührung der Isocyanatkomponente, nach der die Oberfläche des Schaums nicht mehr klebrig ist);

$T_4$ = Zupfzeit (Zeit in Sekunden nach der Einrührung der Isocyanatkomponente, nach der es nicht mehr möglich ist, kleine Stücke mit den Fingern aus dem Schaum zu reißen.

Halbharter Polyurethan-Integralschaum

Halbharter Polyurethan-Integralschaum auf Basis aliphatischer Isocyanate.

Beispiel 8

| | | |
|---|---|---|
| Rezeptur: 1. | 80 g | Polyol A |
| 2. | 7 g | Ethylenglykol |
| 3. | 0,5g | Dibutylzinndilaurat |
| 4. | 12 g | Trichlorfluormethan |
| 5. | x g | amidinischer Co-Katalysator |

Man vermischt die obigen Komponenten 1-5 sorgfältig 30 sec. mit einem Schnellrührer bei Raumtemperatur, gibt dann 41 g Isocyanat B hinzu und verrührt weitere 10 sec. Die Masse wird im Freipäckchen verschäumt.

Die erhaltenen Zeiten sind in Tabelle 1 zusammengefasst.

Tabelle 1: Daten von Versuch 8

| Katalysator | Menge (g) | Zeiten (sec) $T_1$ | $T_4$ |
|---|---|---|---|
| Katalysator B | 1,2 | 43±5 | 128±10 |
| Katalysator C | 0,8 | 46±5 | 110±10 |
| Katalysator D | 0,4 | 19±3 | 98±10 |
| | 0,8 | 10±1 | 55± 5 |
| Katalysator E | 0,4 | 21± 3 | 122±10 |
| | 0,6 | 14± 1 | 95±10 |
| Katalysator G | 0,4 | 22± 3 | 97±10 |
| | 0,6 | 17± 3 | 69± 5 |
| Diazabicycloundecen (zum Vergleich) | 0,4 | 25± 3 | 103±10 |

Beispiel 9

Halbharter Polyurethan-Integralschaum auf Basis aromatischer Isocyanate

| | | |
|---|---|---|
| Rezeptur: | 1. | 70 g Polyol B |
| | 2. | 20 g Polyol C |
| | 3. | 1 g Ethylenglykol |
| | 4. | 14 g Butandiol-1,4 |
| | 5. | 0,1g Wasser |
| | 6. | 10 g Trichlorfluormethan |
| | 7. | 0,02g Dibutylzinndilaurat |
| | 8. | x g amidinischer Co-Katalysator |

Man vermischt die obigen Komponenten sorgfältig 30 sec. mit einem Schnellrührer bei Raumtemperatur, gibt dann 74 g Isocyanat A hinzu und verrührt weitere 10 sec. Die Masse wird im Freipäckchen verschäumt.

Die erhaltenen Zeiten sind in Tabelle 2 zusammengefasst.

Tabelle 2: Daten von Versuch 9

| Katalysator | Menge (g) | Zeiten (sec) $T_1$ | $T_2$ | $T_3$ | $T_4$ |
|---|---|---|---|---|---|
| Katalysator A | 0,8 | 15±1 | 27±3 | 27± 3 | 27±3 |
| Katalysator B | 1,2 | 38±3 | 58±5 | 58±5 | 62±5 |
| | 1,8 | 28±3 | 53±5 | 53±5 | 57±5 |
| Katalysator C | 0,8 | 23±3 | 35±3 | 35±3 | 37±3 |
| | 1,2 | 16±1 | 27±3 | 27±3 | 32±3 |
| Katalysator D | 0,3 | 23±3 | 32±3 | 35±3 | 35±3 |
| | 0,15 | 30±3 | 37±3 | 42±3 | 42±3 |
| Katalysator E | 0,3 | 23±3 | 35±3 | 36±3 | 36±3 |
| | 0,15 | 25±3 | 40±5 | 44±5 | 51±5 |
| Katalysator G | 0,3 | 24±3 | 33±3 | 37±3 | 37±3 |
| | 0,15 | 27±3 | 42±5 | 48±5 | 48±5 |
| Triethylendiamin (DABCO$^{(R)}$) (zum Vergleich) | 0,3 | 25±3 | 38±3 | 50±5 | 62±5 |

Beispiel 10

Polyurethan-Weichschaum

Rezeptur:
1. 50 g Polyol C
2. 1,5g Wasser
3. 0,5g eines handelsüblichen Polysiloxan-Polyalkylenoxid-Blockpolymerisats als Schaumstabilisator
4. 0,05g Zinndioctoat
5. x g Amidin-Katalysator

Man vermischt die obigen Komponenten 30 sec mit einem Schnellmischer bei Raumtemperatur miteinander, gibt 18 g Isocyanat C hinzu und vermischt weitere 5 sec. Die Masse wird im Freipäckchen verschäumt. Die betreffenden Zeiten ($T_1$ = Startzeit, $T_2$ = Steigzeit) sind in Tabelle 3 aufgeführt.

Tabelle 3

| Katalysator (0,3 g) | Startzeit (sec ± 3) | Steigzeit (sec ± 5) |
|---|---|---|
| Katalysator D | 13 | 79 |
| Katalysator E | 13 | 98 |
| Katalysator G | 10 | 73 |
| Vergleich [a] | 14 | 62 |

[a] handelsüblicher aminischer Weichschaumkatalysator auf Basis permethylierter Piperazin- und Diethylentriamin-Verbindungen.

Harter Polyurethan-Integralschaum

Beispiel 11a (Vergleichsbeispiel)

Rezeptur (Komponente A):

1.) 100 g Polyol D
2.) 1 g eines handelsüblichen Polysiloxan-Polyalkylenoxid-Blockcopolymerisats als Schaumstabilisator
3.) 3 g N-Dimethylbenzylamin
4.) 0,5 g Tetramethylguanidin
5.) 3,0 g Amidamin, hergestellt aus 1 Mol 3-Dimethylaminopropylamin-1 und 2 Mol Ölsäure, als inneres Trennmittel
6.) 0,2 g 85%ige wäßrige ortho-Phosphorsäure als Reaktionsverzögerer
7.) 10 g Trichlormonofluormethan als Treibmittel

werden bei Raumtemperatur mit einem Schnellrührer 30 sec miteinander vermischt. Dann gibt man zu 100 g der obigen Mischung Isocyanat D hinzu, vermischt weitere 10 sec mit einem Schnellrührer und gießt dann sofort in eine offene Papierform (Maße: 250 x 120 x 120 mm). Es werden folgende Reaktionszeiten erhalten

Startzeit: 24 ± 3 sec
Abbindezeit: 42 ± 5 sec

Beispiel 11 b:

| | | |
|---|---|---|
| Rezeptur: | 1. | 10 g Polyol D |
| | 2. | 1 g Schaumstabilisator (wie in Beispiel 11 a) |
| | 3. | 3 g Amidamin (wie bei Beisp. 11 a) |
| | 4. | x g Amidin-Katalysator |
| | 5. | 10 g Trichlormonofluormethan |
| | 6. | 1 g Isocyanat D |

Es wird wie bei Beispiel 11 a verfahren. Die erhaltenen Reaktionszeiten sind in Tabelle 5 aufgeführt.

Beispiel 11 c:

In dieser Versuchsreihe werden der Polyolmischung vor dem Vermischen mit dem Amidin-Katalysator 0,2 g 85 %ige wäßrige ortho-Phosphorsäure als Reaktionsverzögerer zugesetzt. Die erhaltenen Reaktionszeiten sind in Tabelle 6 aufgeführt.

Tabelle 4

| Katalysator | Katalysator-Menge (g / Mol) | Startzeit (sec) | Abbindezeit (sec) |
|---|---|---|---|
| Katalysator A | 0,9 g/4,05 mmol | 29±3 | 38±3 |
| | 1,0 g/4,50 mmol | 23±3 | 33±3 |
| | 1,1 g/4,95 mmol | 19±3 | 28±3 |
| Katalysator B | 1,6 g/4,5 mmol | 35±3 | 65±5 |
| | 1,9 g/5,5 mmol | 30±3 | 50±5 |
| | 2,4 g/6,9 mmol | 26±3 | 42±5 |
| Katalysator C | 1,85g/4,5 mmol | 21±3 | 28±3 |
| | 1,7 g/4,15 mmol | 25±3 | 44±5 |
| Katalysator D | 1,25g/4,5 mmol | 14±3 | 18±3 |
| | 0,9 g/3,24mmol | 22±3 | 27±3 |
| | 0,85g/3,06mmol | 25±3 | 30±3 |
| Katalysator E | 1,38g/4,5mmol | 14±3 | 19±3 |
| | 1 g/3,25mmol | 22±3 | 26 ±3 |
| | 0,9 g/2,93mmol | 24±3 | 29±3 |
| Katalysator F | 2,6 g/4,5 mmol | 45±5 | 75±5 |
| | 7 g/12,07mmol | 40±5 | 110±10 |
| Katalysator G | 0,82 g/4,5 mmol | 24±3 | 32±3 |
| 2,5 g Dimethylbenzylamin[a)]<br>0,3 g Tetramethylguanidin | | 24±3 | 37±3 |
| 3 g Dimethylbenzylamin<br>0,5 g Tetramethylguanidin | | 19±3 | 27±3 |
| 2,5 g Dimethylbenzylamin<br>0,25g Tetramethylguanidin | | 24±3 | 43±5 |

a) zum Vergleich

Tabelle 6

| Katalysator A | Menge (g/Mol) | Startzeit (sec) | Abbindezeit (sec) |
|---|---|---|---|
| Katalysator A | 1,3 g/5,9 mmol | 32±3 | 52±5 |
| | 1,5 g/6,76mmol | 24±3 | 38±3 |
| | 1,6 g/7,21mmol | 21±3 | 32±3 |
| Katalysator B | 2,3 g/6,76mmol | 55±5 | 115±10 |
| | 3 g/8,67mmol | 35±3 | 80±5 |
| | 3,5 g/10,12mmol | 25±3 | 45±5 |
| Katalysator C | 2,77g/6,76mmol | 50±5 | 90±10 |
| | 3,5 g/8,54mmol | 23±3 | 32±5 |
| | 4,0 g/9,67mmol | 17±3 | 23±5 |
| Katalysator D | 1,2 g/4,32mmol | 32±3 | 41±3 |
| | 1,35g/4,86mmol | 25±3 | 34±3 |
| | 1,5 g/5,4 mmol | 20±3 | 25±3 |
| | 1,9 g/6,76mmol | 18±3 | 22±3 |
| Katalysator E | 1,5 g/4,89mmol | 25±3 | 34±3 |
| | 1,8 g/5,87mmol | 18±3 | 23±3 |
| | 2,07g/6,76mmol | 16±3 | 22±3 |
| Katalysator F | 3,92 g/6,76mmol | 75±5 | 170±15 |
| | 7,6 g/13,1 mmol | 70±5 | 150±15 |
| Katalysator G | 1,24 g/6,76mmol | 32±3 | 43±5 |
| | 1,5 g/8,2 mmol | 24±3 | 33±3 |

Beispiel 12:

Halbharter Polyurethan-Integralschaum

Dieses Beispiel demonstriert den Selbsttreibeffekt von Katalysator B.

Rezeptur:

1. 70 g Polyol B
2. 20 g Polyol C
3. 1 g Ethylenglykol
4. 14 g Butan-1,4-diol
5. 1 g Katalysator B

Die Komponenten werden bei Raumtemperatur mit einem Schnellmischer sorgfältig 30 sec. verrührt, dann werden 74 g Isocyanat A hinzugegeben, weitere 10 sec. mit einem Schnellmischer verrührt und dann in einer Papierform verschäumt.
Folgende Daten werden erhalten:

$T_1$ = 20 sec. $\pm$ 3; $T_2$, $T_3$, $T_4$ = 40 sec. $\pm$ 3

Die Freischaumdichte beträgt 0,35 $kg/m^3$.

Ansprüche:

1. Tetrahydropyrimidine der allgemeinen Formel

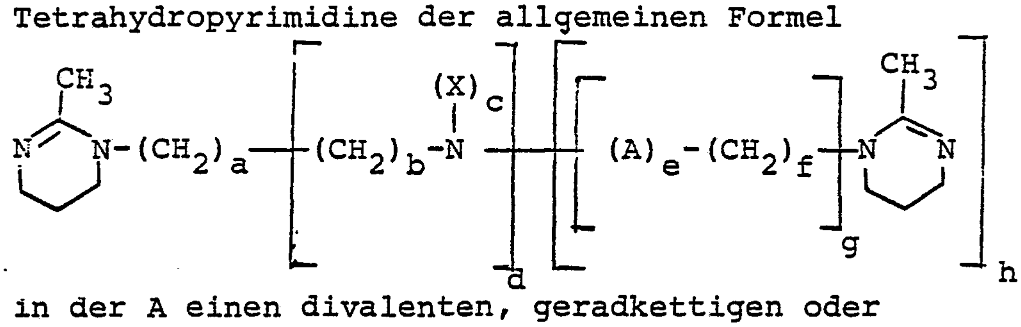

in der A einen divalenten, geradkettigen oder verzweigten $C_1$-$C_{17}$-Alkanrest oder einen divalenten $C_5$-$C_{10}$-Cycloalkanrest oder einen Dicyclohexyl-$C_1$-$C_4$-alkanrest, die durch einen Phenylrest, eine Cyangruppe oder einen Halogenrest substituiert sein können und wobei der $C_1$-$C_{17}$-Alkanrest in der Kette durch -NH-COO-, -O- und/oder -S-Einheiten unterbrochen sein kann,

X einen geradkettigen oder verzweigten $C_1$-$C_{16}$-Alkylrest oder einen $C_5$-$C_7$-Cycloalkylrest, die durch einen Phenylrest, eine Cyangruppe, eine Hydroxygruppe oder einen Halogenrest substituiert sein können, oder, wenn h = 0 ist, zusammen einen Ring mit zusammen 3-6 Ring-C-Atomen bilden, von denen ein oder zwei C-Atome durch -O-, -S-, -NH- oder $-NR^3-$ ersetzt sein können, wobei $R^3$ für $C_1$-$C_6$-Alkyl oder ω-Hydroxy-$C_1$-$C_6$-Alkyl steht,

a, b, f, g jeweils ganze Zahlen von 1 bis 16,
c 1 oder 2,
h 0 oder 1,
wobei $\sum c + h = 2$ ist
d 0 oder eine ganze Zahl von 1 bis 10 und
e 0 oder 1
bedeuten.

2. Tetrahydropyrimidine gemäß Anspruch 1 der allgemeinen Formel

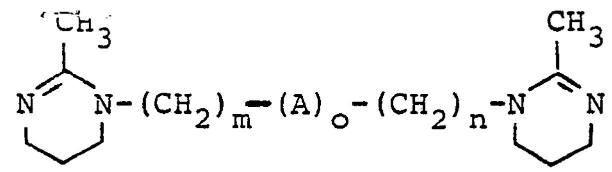

in der A einen divalenten, geradkettigen oder verzweigten $C_1$-$C_{17}$-Alkanrest oder einen divalenten $C_5$-$C_{10}$-Cycloalkanrest oder einen Dicyclohexyl-$C_1$-$C_4$-alkanrest, die durch einen Phenylrest, eine Cyangruppe oder einen Halogenrest substitutiert sein können und wobei der $C_1$-$C_{17}$-Alkanrest in der Kette durch -NH-COO-, -O- und/oder -S-Einheiten unterbrochen sein kann,

m eine ganze Zahl von 1 bis 16,

n eine ganze Zahl von 1 bis 16 und

o 0 oder 1 bedeuten.

3. Tetrahydropyrimidine gemäß Anspruch 1 der allgemeinen Formel

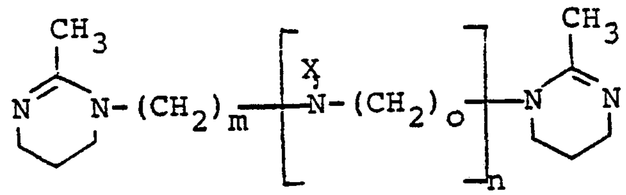

in der X einen geradkettigen oder verzweigten $C_1$-$C_{16}$-Alkylrest oder einen $C_5$-$C_7$-Cycloalkylrest, die durch einen Phenylrest, eine Cyangruppe oder einen Halogenrest substituiert sein können,

m, o jeweils eine ganze Zahl von 2 bis 10 und

n eine ganze Zahl von 1 bis 16

bedeuten.

4. Tetrahydropyrimidine gemäß Anspruch 1 der allgemeinen Formel

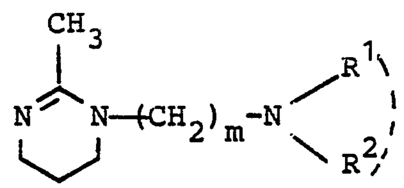

,

in der

m eine ganze Zahl von 2 bis 10 ist und

$R^1$,$R^2$ unabhängig voneinander einen geradkettigen oder verzweigten $C_1$-$C_{10}$-Alkylrest oder einen $C_5$-$C_7$-Cycloalkylrest, die durch einen Phenylrest, eine Cyangruppe, eine Hydroxylgruppe oder ein Halogenatom substituiert sein können, bedeuten, oder zusammen einen Ring mit zusammen 3-6-Ring-C-Atomen bilden, von denen ein oder zwei C-Atome durch -O-, -S-, -NH- oder -$NR^3$- ersetzt sein können, wobei

$R^3$ für $C_1$-$C_6$-Alkyl oder ω-Hydroxy-$C_1$-$C_6$-alkyl steht.

5. Tetrahydropyrimidine gemäß Anspruch 1 bis 4 in Form ihrer Salze, Additionsverbindungen und Komplexe mit Lewis- und Protonensäuren.

0054876

6. Verfahren zur Herstellung von Tetrahydropyrimidinen der allgemeinen Formel

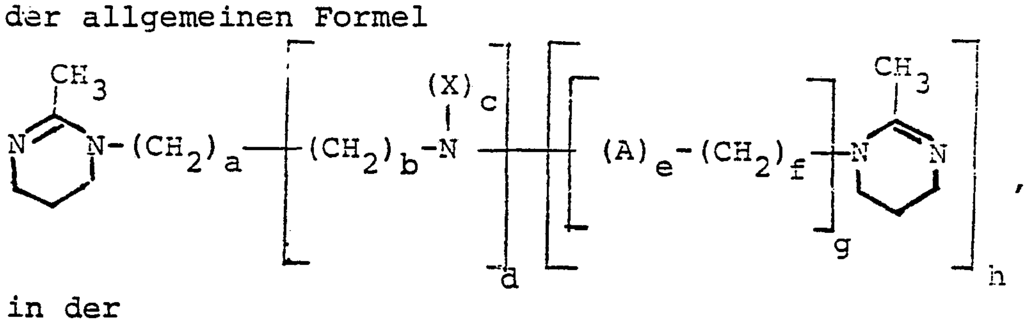

in der

A einen divalenten geradkettigen oder verzweigten $C_1$-$C_{17}$-Alkanrest oder einen divalenten $C_5$-$C_{10}$-Cycloalkanrest oder einen Dicyclohexyl-$C_1$-$C_4$-alkanrest, die durch einen Phenylrest, eine Cyangruppe oder einen Halogenrest substituiert sein können und wobei der $C_1$-$C_{17}$-Alkanrest in der Kette durch -NH-COO-, -O- und/oder -S-Einheiten unterbrochen sein kann,

X einen geradkettigen oder verzweigten $C_1$-$C_{16}$-Alkylrest oder einen $C_5$-$C_7$-Cycloalkylrest, die durch einen Phenylrest, eine Cyangruppe, eine Hydroxygruppe oder einen Halogenrest substituiert sein können, oder, wenn h = 0 ist, zusammen einen Ring mit zusammen 3-6 Ring-C-Atomen bilden, von denen ein oder zwei C-Atome durch -O-, -S-, -NH- oder -$NR^3$- ersetzt sein können, wobei $R^3$ für $C_1$-$C_6$-Alkyl oder $\omega$-Hydroxy-$C_1$-$C_6$-Alkyl steht,

a, b, f, g jeweils ganze Zahlen von 1 bis 16,

c 1 oder 2,

h 0 oder 1,

wobei $\sum c + h = 2$ ist,

d 0 oder eine ganze Zahl von 1 bis 10 und

e 0 oder 1

bedeuten,
durch Umsetzung von N-substituierten Propylendiaminen und Acetessigsäurederivaten, dadurch gekennzeichnet, daß man als N-substituierte Bispropylendiamine Verbindungen der allgemeinen Formel

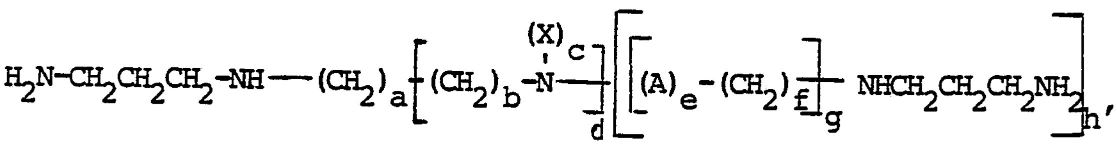,

in der
A, X, a, b, c, d, e, f, g und h die oben angegebenen Bedeutungen haben,

mit einem Acetessigsäurederivat der allgemeinen Formel

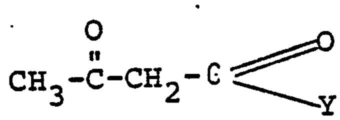 ,

in der
Y für $-OR^4$, $-NHR^4$ oder $-NR^4_2$ und

$R^4$ für einen $C_6$-$C_{10}$-Aryl-, $C_1$-$C_{17}$-Alkyl- oder $C_5$-$C_7$-Cycloalkylrest

steht, in Abwesenheit von Katalysatoren bei einer Reaktionstemperatur von 0-80°C umsetzt und dabei das bei der Reaktion entstehende Wasser kontinuierlich entfernt.

7. Verfahren zur Herstellung von Tetrahydropyrimidinen der allgemeinen Formel

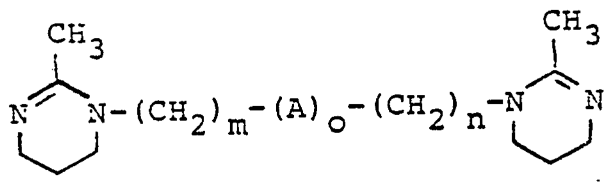

in der A einen divalenten, geradkettigen oder verzweigten $C_1$-$C_{17}$-Alkanrest oder einen divalenten $C_5$-$C_{10}$-Cycloalkanrest oder einen Dicyclohexyl-$C_1$-$C_4$-alkanrest, die durch einen Phenylrest, eine Cyangruppe oder einen Halogenrest substituiert sein können und wobei der $C_1$-$C_{17}$-Alkanrest in der Kette durch -NH-COO-, -O- und/oder -S-Einheiten unterbrochen sein kann,

m eine ganze Zahl von 1 bis 16,
n eine ganze Zahl von 1 bis 16 und
o 0 oder 1 bedeuten, durch Umsetzung von N-substituierten Bis-propylendiaminen mit Acetessigsäurederivaten, dadurch gekennzeichnet, daß man als N-substituierte Bis-propylendiamine Verbindungen der allgemeinen Formel

$$H_2NCH_2CH_2CH_2NH(CH_2)_m-(A)_o-(CH_2)_n-NHCH_2CH_2CH_2NH_2 ,$$

in der A, m, n und o die oben genannten Bedeutungen haben, mit einem Acetessigsäurederivat

der allgemeinen Formel

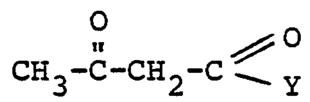

in der
Y für $-OR^4$, $-NHR^4$ oder $-NR^4_2$ und
$R^4$ für einen $C_6$-$C_{10}$-Aryl-, $C_1$-$C_{17}$-Alkyl- oder $C_5$-$C_7$-Cycloalkylrest steht,
in Abwesenheit von Katalysatoren bei einer Reaktionstemperatur von 0-80°C umsetzt und dabei das bei der Reaktion entstehende Wasser kontinuierlich entfernt.

8. Verfahren zur Herstellung von Tetrahydropyrimidinen der allgemeinen Formel

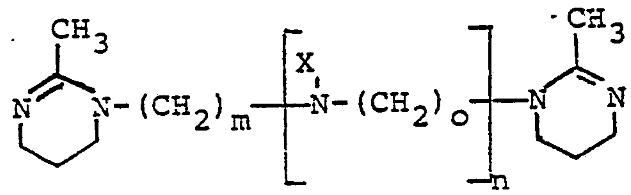
,

in der
X einen geradkettigen oder verzweigten $C_1$-$C_{16}$-Alkylrest oder einen $C_5$-$C_7$-Cycloalkylrest, die durch einen Phenylrest, eine Cyangruppe oder einen Halogenrest substituiert sein können,
m, o jeweils eine ganze Zahl von 2 bis 10 und
n eine ganze Zahl von 1 bis 16 bedeuten,
durch Umsetzung von N-substituierten Bis-propylendiaminen mit Acetessigsäurederivaten, dadurch gekennzeichnet, daß man als N-substituierte

Bis-propylendiamine Verbindungen der allgemeinen Formel

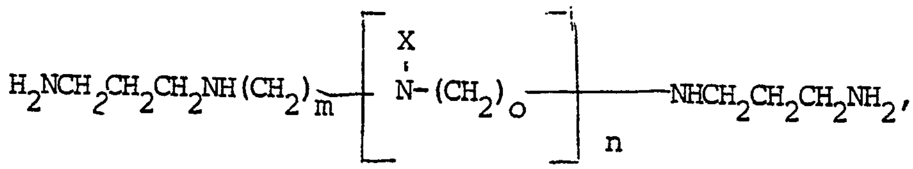

in der X, m, n und o die oben angegebene Bedeutung haben, mit einem Acetessigsäurederivat der allgemeinen Formel

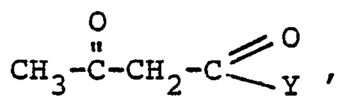

in der
Y für eine $-OR^4$, $-NHR^4$ oder $-NR^4_2$ und
$R^4$ für einen $C_6$-$C_{10}$-Aryl-, $C_1$-$C_{17}$-Alkyl- oder $C_5$-$C_7$-Cycloalkylrest steht,
in Abwesenheit von Katalysatoren bei einer Reaktionstemperatur von 0-80°C umsetzt und dabei das bei der Reaktion entstehende Wasser kontinuierlich entfernt.

9. Verfahren zur Herstellung von Tetrahydropyrimidinen der allgemeinen Formel

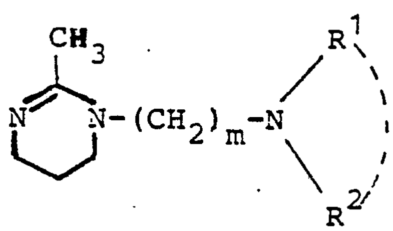

in der
m eine ganze Zahl von 2 bis 10 ist und
$R^1$, $R^2$ unabhängig voneinander einen geradkettigen, oder verzweigten $C_1$-$C_{10}$-Alkylrest oder einen $C_5$-$C_7$-Cycloalkylrest, die durch einen Phenylrest, eine

Cyan- oder Hydroxylgruppe oder ein Halogenatom substituiert sein können, bedeuten, oder zusammen einen Ring mit zusammen 3-6 Ring-C-Atomen bilden, von denen ein oder zwei C-Atome durch -O-, -S-, -NH- oder $-NR^3-$ ersetzt sein können, wobei

$R^3$ für $C_1$-$C_6$-Alkyl oder $\omega$-Hydroxy-$C_1$-$C_6$-alkyl steht,

durch Umsetzung von N-substituierten Bis-propylendiaminen und Acetessigsäurederivaten, dadurch gekennzeichnet, daß man als N-substituierte Bispropylendiamine Verbindungen der allgemeinen Formel

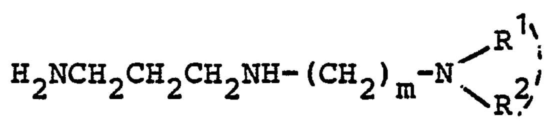 ,

in der m, $R^1$ und $R^2$ die oben genannten Bedeutungen haben, mit einem Acetessigsäurederivat der allgemeinen Formel

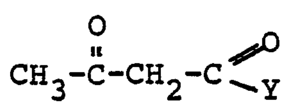 ,

in der

Y für $-OR^4$, $-NHR^4$ oder $-NR^4_2$ und $R^4$ für einen $C_6$-$C_{10}$-Aryl-, $C_1$-$C_{17}$-Alkyl- oder $C_5$-$C_7$-Cycloalkylrest steht, in Abwesenheit von Katalysatoren bei einer Reaktionstemperatur von 0-80°C umsetzt und dabei das bei der Reaktion entstehende Wasser kontinuierlich entfernt.

10. Verwendung der Tetrahydropyrimidine gemäß Anspruch 1 bis 5 als Katalysatoren bei der Herstellung von Polyurethankunststoffen durch Umsetzung von Polyisocyanaten mit mindestens zwei gegenüber Isocyanaten aktive Wasserstoffatome aufweisenden Verbindungen vom Molekulargewicht 400 bis 10 000, gegebenenfalls in Gegenwart von Verbindungen mit mindestens zwei gegenüber Isocyanaten reaktionsfähigen Wasserstoffatomen und einem Molekulargewicht von 32 bis 400 als Kettenverlängerungsmittel und gegebenenfalls in Gegenwart von Treibmitteln und weiteren Hilfs- und Zusatzstoffen.

0054876

Europäisches Patentamt

# EUROPÄISCHER RECHERCHENBERICHT

Nummer der Anmeldung

EP 81110409.0

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| D,A | DE - A1 - 2 737 671 (BAYER)<br>* Patentansprüche 1,3 *<br>-- | 1,10 | C 07 D 239/06<br>C 08 G 18/20 |
| A | US - A - 3 549 592 (GODFREY)<br>* Patentansprüche 1,4,5 *<br>---- | 1 | |

RECHERCHIERTE SACHGEBIETE (Int. Cl.³)

C 07 D 239/00
C 08 G

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 30-03-1982 | HAMMER |

EPA form 1503.1 06.78